# EUROPEAN PATENT SPECIFICATION

(11) **EP 3 752 213 B1**
(45) Date of publication and mention of the grant of the patent: **25.02.2026**
(21) Application number: 19764862.9
(22) Date of filing: 05.03.2019
(51) Int. Cl.: A61M 1/00, A61M 27/00

(54) **DEVICES AND METHODS FOR MANAGING CHEST DRAINAGE**
VORRICHTUNGEN UND VERFAHREN ZUR VERWALTUNG VON THORAXDRAINAGE
DISPOSITIFS ET PROCÉDÉS DE GESTION DU DRAINAGE THORACIQUE

(30) Priority: 06.03.2018 US 201862639326 P; 07.09.2018 US 201862728585 P; 29.01.2019 US 201962798379 P
(43) Date of publication of application: 23.12.2020
(73) Proprietor: Centese, Inc., Omaha, NE 68117 (US)
(72) Inventor: LUXON, Evan S., Omaha, Nebraska 68112 (US); COUGHLIN, Ryan, Omaha, Nebraska 68114 (US); BEHRINGER, Ryan, Omaha, Nebraska 68131 (US); FITCH, Kyle, Omaha, Nebraska 68105 (US); PRESTON, Randy, San Francisco, California 94118 (US); BURNETT, Daniel R., San Francisco, California 94109 (US); ZIEGLER, Mark, Palo Alto, California 94301 (US)
(74) Representative: Lorenz Seidler Gossel Part. mbB
(86) International application number: PCT/US2019/020809
(87) International publication number: WO 2019/173379

(56) References cited:
- WO-A1-2007/024230
- WO-A1-2016/054051
- WO-A2-2017/152125
- WO-A2-2017/152125
- US-A1- 2007 078 444
- US-A1- 2007 078 444
- US-A1- 2014 213 992
- US-A1- 2016 193 393
- US-A1- 2017 354 768
- US-A1- 2017 354 777
- US-A1- 2018 056 050

## Description

### FIELD OF THE INVENTION

The present invention relates to wound and surgical drainage.

### BACKGROUND OF THE INVENTION

Chest tubes are required any time air and/or liquid accumulates in the chest cavity, disrupting normal pulmonary or cardiac function. Suction is commonly applied continuously to remove excess air and/or fluid from the chest until the internal wounds have healed, at which point the chest tubes can be removed. One of the most common uses of chest tubes is to drain the area around the heart after cardiac surgery.

A drainage system for draining excess fluid from the chest is known from, e.g., WO 2017/152125 A2 which suggests to activate a pump in response to a pressure signal from a pressure sensor indicating a suction level sufficient to drain a fluid from the chest tube drainage lumen.

Despite their benefits, current chest tube systems suffer from two major flaws. First, as liquid drains from the chest toward the suction container, it can pool in the drainage tubing and prevent the applied negative pressure from being transmitted to the chest. When this occurs, the pressure in the chest can be reduced to zero or even become positive, preventing proper drainage. Second, clogs can form in the chest tube which can obstruct the chest tube, which prevents the negative pressure from being transmitted to the chest and inhibits drainage. In fact, 36% of cardiac surgery patients experience chest tube clogging. When proper drainage is inhibited due to these factors, patients are at increased risk for accumulation of fluid around the heart, known as pericardial tamponade, which results in shock and can be fatal. Additionally, the lungs may be compressed, which can lead to respiratory compromise and can be fatal as well.

Pooling of liquid in the drainage line can theoretically be remedied by keeping the tubing straight from the patient to the collection container. However, this is nearly impossible in practice, as some slack is required to prevent accidental dislodging of the tube from the body. To combat clogging, clinicians use two methods known as milking and stripping. Milking refers to line manipulations such as lifting, squeezing, or kneading. Stripping refers to a pulling along the length of the tube with the thumb and forefinger to increase the amount of suction at the end of the tube. However, these methods have not been shown to be effective at improving chest tube suction or drainage. In fact, stripping has actually been discouraged because it is possible to create extremely high negative pressures (up to -370 cmH2O) that may damage the tissue.

In addition to these functional flaws, current systems also measure collected fluid volume and rate of chest air leak inaccurately and/or subjectively. As a result, clinicians make cautious clinical decisions based on these measurements, keeping patients in the hospital longer than necessary.

### SUMMARY OF THE INVENTION

A chest drainage system is needed which reduces or eliminates pooling of blood/liquid and/or clogging/clotting in the drainage tube and/or chest tube, and provides objective and accurate measures of collected fluid volume and chest/thoracic air leak.

To address at least some of the aforementioned deficiencies of prior art devices, the present invention suggests a drainage system as defined by claim 1. Preferred embodiments of the invention are laid down in the dependent claims.

In one embodiment, a drainage system may generally comprise a chest tube having a chest tube drainage lumen and a drainage reservoir in fluid communication with the chest tube drainage lumen. A pump may be in fluid communication with the chest tube drainage lumen and a pressure sensor may be positioned proximal to the chest tube and in communication with the chest tube drainage lumen. Furthermore, a controller is in communication with the pressure sensor and the pump, wherein the controller is configured to actuate the pump at a first suction level sufficient to drain a fluid from the chest tube drainage lumen, and wherein the controller is further configured to actuate the pump at a second suction level which is higher than the first suction level such that an absence of attenuation in the second suction level over time is indicative of an obstruction in the chest tube.

The drainage system, in functional respect, may generally comprise receiving a fluid through a chest tube having a chest tube drainage lumen, and applying a first suction level to the chest tube drainage lumen sufficient to drain the fluid from the chest tube. A pressure may be monitored within a drainage pathway from the chest tube drainage lumen via a pressure sensor in communication with a controller. Furthermore, a second suction level may be applied to the chest tube drainage lumen which is different from the first suction level such that an absence of attenuation in the second suction level over time is indicative of an obstruction in the chest tube.

### BRIEF DESCRIPTION OF THE DRAWINGS

Fig. 1 shows an embodiment of the chest drainage system.
Fig. 2 shows various components of the chest drainage system.
Fig. 3 shows an enlarged view of the chest tube.
Fig. 4 shows an enlarge view of the chest tube relief valve.
Fig. 5 shows an enlarged view of the drainage tube and the canister.
Figs. 6A and 6B shows a diagram of an embodiment of the chest tube.
Fig. 7 shows a magnetic embodiment of the chest tube relief valve.
Fig. 8A shows the chest drainage system's ability to detect and clear pooled liquid in the drainage tube.
Figs. 8B-8I show the chest drainage system's ability to detect and clear pooled liquid in the chest tube.
Figs. 9, 10, 11 and 12 show an embodiment of a dual-lumen chest tube.
Fig. 13 shows an embodiment of a dual lumen chest tube.
Fig. 14 shows an embodiment of the controller/monitor and the collection canister.
Fig. 15 shows an embodiment of a collection reservoir/canister.
Fig. 16 shows a latching mechanism between the canister/reservoir and the monitor.
Fig. 17 shows a modular attachment.
Fig. 18 shows an embodiment of a connection barb.
Figs. 19A-19Z show possible graphic user interface screens.
Fig. 20 shoes an embodiment of the chest tube which includes a sheath.
Fig. 21 shows an embodiment of the chest tube which can be cut to length.
Fig. 22 shows an embodiment of the system which combines more than one chest tube.
Fig. 23 shoes an embodiment of the chest tube which includes a sheath.
Fig. 24 shoes an embodiment of the chest tube which includes heat shrink tubing.
Fig. 25 shoes an embodiment of the chest tube which includes heat shrink tubing.
Fig. 26 shoes an embodiment of the chest tube in which the drainage holes are not completely punched through.
Fig. 27 shoes an embodiment of the chest tube which includes a drainage channel opening.
Fig. 28 shoes an embodiment of the chest tube which includes a drainage channel opening.
Fig. 29 shoes an embodiment of the chest tube which includes a drainage channel opening.
Fig. 30 shoes an embodiment of the chest tube which includes more than one drainage channel opening.
Fig. 31 shows a repellant barrier.
Fig. 32 shows an embodiment of the drainage canister with more than one collection chamber.
Fig. 33 shows an embodiment of the drainage canister with more than one collection chamber.
Fig. 34 shows some common alarm codes.
Fig. 35 shows an embodiment of the chest tube which includes sensors.
Fig 36 is a block diagram of a data processing system.

### DETAILED DESCRIPTION OF THE INVENTION

Disclosed is a chest drainage system which reduces or eliminates pooling of blood/liquid and/or clogging/clotting in the drainage tube and/or chest tube, and provides objective and accurate measures of drained fluid volume and chest air leak.

The chest drainage system continuously monitors chest tube and drainage tube status and clears pooled liquid in the drainage tube, and/or a clogged chest tube when necessary to restore negative pressure to the chest, allowing it to drain. The system may include active and/or passive valve functions, as well as a controller (also referred to herein as a monitor) for monitoring the pressures in the system. The controller may control a pump for assisting in clearance of pooled liquid and/or clots in the drainage tube and/or chest tube. The controller may also control any active valves and/or suction device in response to measured pressure signals. The controller may also measure and communicate collected fluid volume and air leak information. The chest drainage system performs at least six primary functions:

### Functions:

### 1. Drainage tube blockage detection

The chest drainage system detects pooled liquid in the drainage tube by monitoring the pressure at or near the chest tube-drainage tube interface (the tube-tube interface or junction area). Pooled liquid in the drainage tube is indicated by a decrease in vacuum (increasing pressure) at the tube-tube interface. The chest drainage system may measure pressure with a sensor incorporated into the controller. The sensor may be in fluid communication with the tube-tube interface area via a fluid filled lumen (the relief lumen). The relief lumen may be open to atmosphere on the other end, and be filled with air. A valve (drainage tube valve, drainage tube relief valve or drainage tube relief lumen valve) may be used to open the relief lumen, exposing the tube-tube interface to atmospheric pressure. The drainage tube relief valve may also close the relief lumen, and may include a vent which prevents the transmission of bacteria and viruses from the atmosphere into the relief lumen. The drainage tube relief valve may be opened and closed by the controller based on the measured pressure at the tube-tube interface area.

Alternatively, the pressure sensor may be placed at the tube-tube interface area, connected directly to atmosphere. In this embodiment, the pressure sensor is in communication with the controller. Alternatively, the drainage tube relief valve may be passive, either with or without a relief lumen. Alternatively, the drainage tube relief valve may be operated manually.

Alternatively or additionally, a pressure sensor may be placed in the chest fluid collection chamber/reservoir/cassette/receptacle/canister. At a time when the vacuum pump is running, whether to perform a chest tube clearance, a drainage line clearance, or simply to regulate the suction, pressure in the canister may become more negative if a clog in the drainage line is present. A preset pressure (vacuum) threshold may be set by the user or the controller, the exceeding of which, indicates a blocked drainage lumen.

Alternatively, pressure sensors may be present both in the canister and at or around the tube-tube interface. The controller may continuously monitor the pressure differential between these two pressure sensors and detect a blocked drainage tube if the difference exceeds a preset threshold for the pressure difference.

### 2. Drainage tube blockage clearance

When a blocked drainage line is detected (or at timed intervals), the chest drainage system clears the drainage tube by opening the drainage tube relief lumen valve which is in fluid communication with the tube-tube interface area. Opening the drainage tube relief lumen valve allows air to sweep away the liquid, and any blockage, in the drainage tube into the drainage container/reservoir. A pump which may be integrated with the controller, applies negative pressure to the drainage tube (via a collection reservoir/cassette/chamber). Optionally the pump may also apply positive pressure to the relief lumen (rather than its being open to atmospheric pressure) to help clear the blockage. Proper negative pressure at the chest is then restored. Optionally, the system may apply negative pressure (or an increased negative pressure) to the drainage tube without opening the relief lumen valve, or in addition to opening the relief lumen valve to restore proper suction. This measure may be performed when the controller senses a blockage in the drainage tube, or may be performed at set intervals. Pressure measured at the tube-tube interface may drive the controller to initiate a drainage tube clearance cycle The pressure measured at the tube-tube interface may also or alternatively drive the pump so that a desired suction level is maintained at the tube-tube interface during a clearance cycle.

### 3. Chest Tube Blockage Detection

Clots or clogs may form in the chest tube. To detect a blocked chest tube, the controller pulls suction intermittently to a level that exceeds the crack pressure of the chest tube relief valve. Once it hits this first pre-set threshold the pump turns off or reduces the suction (to a more positive pressure level). At this point, if there is no blockage, or if the blockage is able to be cleared via the transfer of the increased suction, the valve will open and the measured vacuum will attenuate down to a second pre-set threshold (more positive than the first threshold). If the controller does not sense this attenuation over a specified time interval, the controller determines that the chest tube is blocked and may issue an alarm, or attempt alternative measures of clearing the chest tube, such as increasing the suction applied to the chest tube to a third threshold. Pressure may be measured at the tube-tube junction or in the canister or elsewhere in the system.

### 4. Chest tube blockage clearance

To clear blockages in the chest tube, the suction magnitude applied at the tube-tube interface may be increased by the controller. A passive chest tube relief valve, in fluid communication with a chest tube relief lumen, may be configured to open when the pressure in the tube-tube interface drops below a set level. The chest tube relief valve may be open to atmospheric pressure and include a filter or vent to prevent bacteria etc. from entering the system. Once the chest tube relief valve is open, the chest tube will be cleared. The chest tube relief valve may be configured to close at a pressure differential which is less than that of the opening pressure, to ensure the valve stays open long enough for the chest tube to be cleared and to minimize the flow resistance of the valve. Alternatively, the chest tube relief valve, may be an active valve, which the controller opens and closes based on pressures measured in the tube-tube interface area and/or in the chest tube relief lumen. An active chest tube relief valve may open and close at the same pressure differential or open and close at different pressure differentials.

Alternatively, the chest tube relief valve may be connected to the drainage line relief lumen and either controlled directly by the controller via a connection to the controller, or via pressure changes introduced through the drainage line relief lumen by the controller.

In some embodiments, one or more of the valves are passive and set to open at a set pressure and stay open until the same, or another, set pressure is reached. In some embodiments, one or more of the valves are active and directly controlled by the controller. In either case, one or more valves may be set to open at one pressure, and close at another pressure.

The intervals for clearing the chest tube may be different than the intervals for clearing the drainage line.

### 5. Chest air leak detection

To detect air leaks from the patient's chest, the controller monitors the flow of air pumped from the canister to maintain the prescribed level of suction within the canister. This is done with a flow meter and/or measuring the revolutions of the pump necessary to evacuate the air.

### 6. Drainage fluid volume measurement

The controller may measure the volume (or flow) of drained chest fluids that is collected within the canister. Collected fluid volume measurements are preferably made with a non-contact capacitive sensor, but may alternatively be made with optical sensors, pressure sensors, acoustic (such as ultrasonic) sensors, a camera, or any other liquid level sensing methods known in the art.

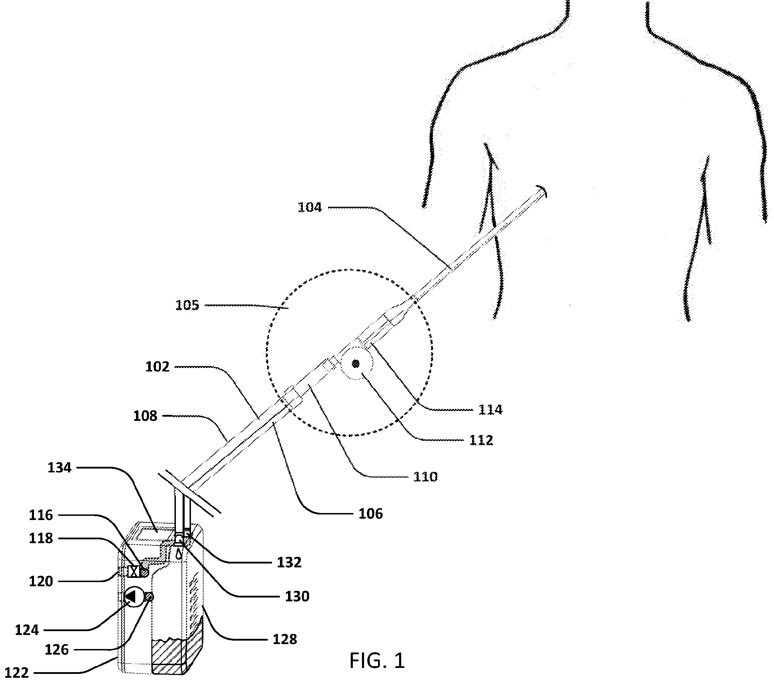

Fig. 1 shows an embodiment of the chest drainage system with an active drainage tube relief valve and a passive chest tube relief valve. Chest tube 104 is connected to, and in fluid communication with, drainage tube 108. Chest tube 104 includes both a chest tube drainage lumen and a chest tube relief lumen. Drainage tube 108 includes both drainage tube drainage lumen 102 and drainage tube relief lumen 106. Drainage tube relief lumen 106 is in fluid communication with drainage tube drainage lumen 102. Drainage tube drainage lumen 102 is in fluid communication with the chest tube drainage lumen. The connection among the 3 lumens - chest tube drainage lumen, drainage tube drainage lumen and drainage tube relief lumen, occurs at or near tube-tube junction 105, which is at or near the chest tube/drainage tube junction. In some embodiments, the drainage tube relief lumen may connect to the drainage tube or chest tube at a different location. The chest tube, drainage tube and drainage tube relief lumen may be connected with drainage tube connection barb 110.

In some embodiments, the drainage tube relief lumen may be in fluid communication with the chest tube relief lumen.

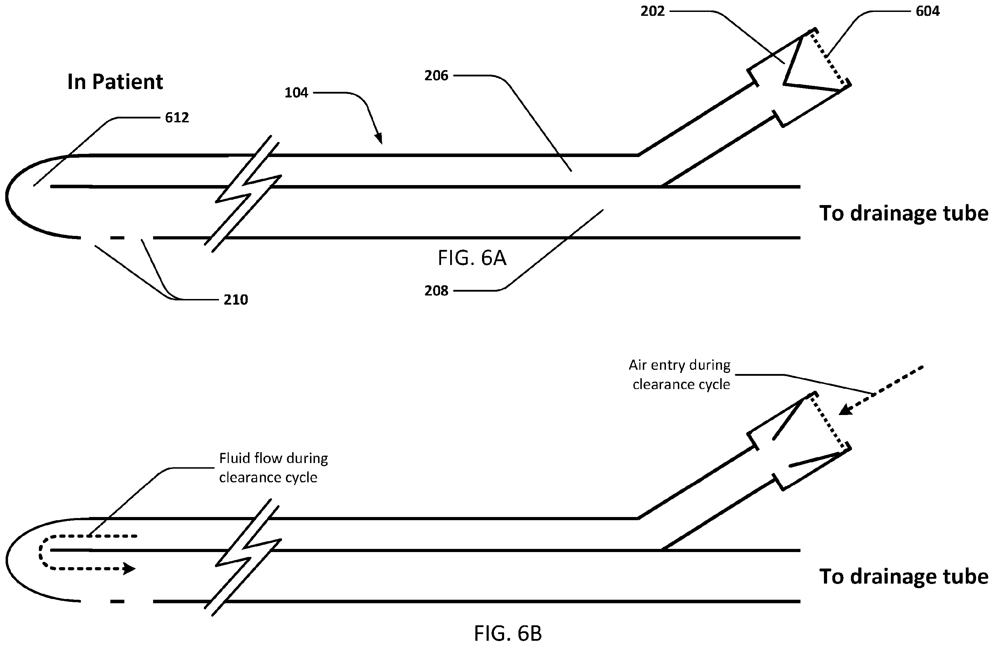

Chest tube relief valve 112 may be incorporated into the chest tube, or a separate adapter designed to connect to the chest tube, for example, into chest tube connection barb 114. In this embodiment, the chest tube has at least two lumens, chest tube drainage lumen and chest tube relief lumen, as shown in Figs. 6A and 6B. Pressure sensor 116, drainage tube relief lumen valve 118, and filter/vent 120 are in fluid communication with drainage tube relief lumen 106.

Controller 122 may include pump 124, pressure sensor 116, drainage tube relief lumen valve 118, filter/vent 120 (which may be on either side of valve 118 and pressure sensor 116), and fluid reservoir (or suction canister) 128, which is in fluid communication with drainage tube 108 via drainage tube drainage lumen connector 130 and drainage tube relief lumen connector 132. However, the drainage line relief lumen may connect to the controller directly, without connecting through the canister. The controller may also include display 134, which may receive input, for example via a touch screen, in addition to displaying information.

Controller 122 may include a suction device, such as pump 124 to create a negative pressure, or suction, force on the drainage tube (possibly via collection canister 128) which is in fluid communication with the chest tube and the chest tube relief valve. In this way, suction may be maintained on the chest cavity to promote chest fluid drainage and aid with patient breathing. The mechanism for creating the negative pressure may be a pump or any other suitable mechanism. The controller and the suction device may be incorporated or may be separate. Any communication between the controller and the suction device and/or any of the valves may be wired or wireless.

Controller 122 may also include pressure sensor 126 on the canister side of the pump, to measure and/or monitor the pressure within the canister. The controller may also include a flow sensor or flow meter on either side of the pump, and/or one-way valve on either side of the pump to measure air/gas pumped out of the canister. The air flow may also or alternatively be measured by measuring the pump revolutions.

Pressure sensor 116 senses the pressure in tube-tube interface area 105 (via drainage tube relief lumen 106). When the drainage tube is blocked or restricted, the pressure in the tube-tube interface area increases. When this pressure increases to a set pressure (generally, a negative pressure), controller 122 opens drainage tube relief valve 118 (which is normally closed) to allow filtered atmospheric pressure air to enter drainage tube relief lumen 106. This influx of air, in combination with the negative pressure in the drainage tube caused by pump 124, acts to clear the drainage tube of blockages/restrictions. Once the pressure in the tube-tube interface area returns to normal, and/or after a set time, the controller closes drainage tube relief valve 118. Alternatively, the drainage tube valve may be a passive valve set to open and close at set pressures.

Alternatively, the controller may be configured to open the drainage tube relief valve periodically, regardless of the pressure measured in the tube-tube interface.

The monitor/controller may monitor pressure in the drainage tube relief lumen and may pull additional suction in the fluid reservoir/suction canister as needed to maintain the suction pressure in the proper range at the tube-tube interface area. For example, when the desired pressure is set to -20 cmH2O, the monitor may activate the suction pump to keep the pressure at the tube-tube interface area between -15 cmH2O and -25 cmH2O or between -18 cmH2O and -22 cmH2O. In another embodiment, the monitor may activate the pump and drainage tube relief valve 118 at regular temporal intervals as a preventative measure to clear any pooled liquid from the drainage line. This is done by the controller activating suction pump 124 while simultaneously opening drainage tube relief valve 118 to allow air to sweep accumulated liquid into the suction canister via the drainage tube.

The chest tube may become blocked or restricted. To clear restrictions, the suction magnitude applied by the controller to the drainage tube and experienced by the tube-tube interface may be increased. When the pressure in the tube-tube interface reaches a set low level (i.e. high level of suction), chest tube relief valve 112 opens and allows filtered atmospheric air to enter the relief lumen of the chest tube (see Figs. 6A and 6B for detail). This influx of air, in combination with the negative pressure in the drainage tube and tube-tube interface area caused by pump 124, acts to clear the chest tube of blockages/restrictions. In instances where a chest tube blockage cannot be cleared, an alarm may sound. A passive valve is shown here, although an active valve, directly controlled by the controller, may be used. Alternatively, a valve which is operated manually, may be used. Any of the operations disclosed herein which may be controlled by the controller, may alternatively be controlled passively, or manually. For example, valve functions, suction functions, etc.

The chest tube relief valve may have a different opening pressure and closing pressure. For example, the chest tube relief valve may open at a higher pressure differential (i.e. a more negative pressure in the tube-tube interface area), and close at a lower pressure differential. This allows the valve to stay closed until a clear chest tube blockage is present and to minimize the flow resistance of the valve. Once the valve is open, this allows the valve to stay open to completely clear the chest tube blockage, even if the tube-tube interface area pressure increases so that the pressure differential across the chest tube valve drops below the valve opening pressure. In other words, the pressure within the tube-tube interface area may be more negative when a chest tube blockage is created, but less negative, as the chest tube blockage is being cleared.

Fig. 1 shows one chest tube in use with the chest drainage system, but in some embodiments, more than one chest tube may be used with the system. Each chest tube may have its own drainage lumen and relief lumen and valve, or they may share a relief lumen and/or valve.

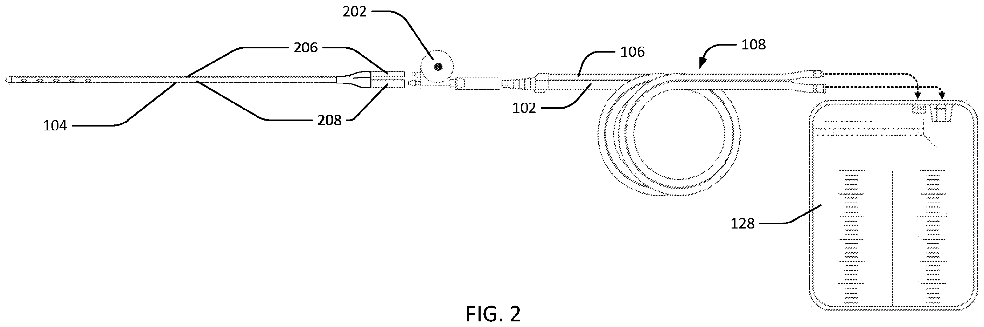

Fig. 2 shows various components of the chest drainage system, including chest tube 104, chest tube relief lumen 206, chest tube drainage lumen 208, chest tube relief valve 202, drainage tube 108, drainage tube relief lumen 106, drainage tube drainage lumen 102, and canister 128.

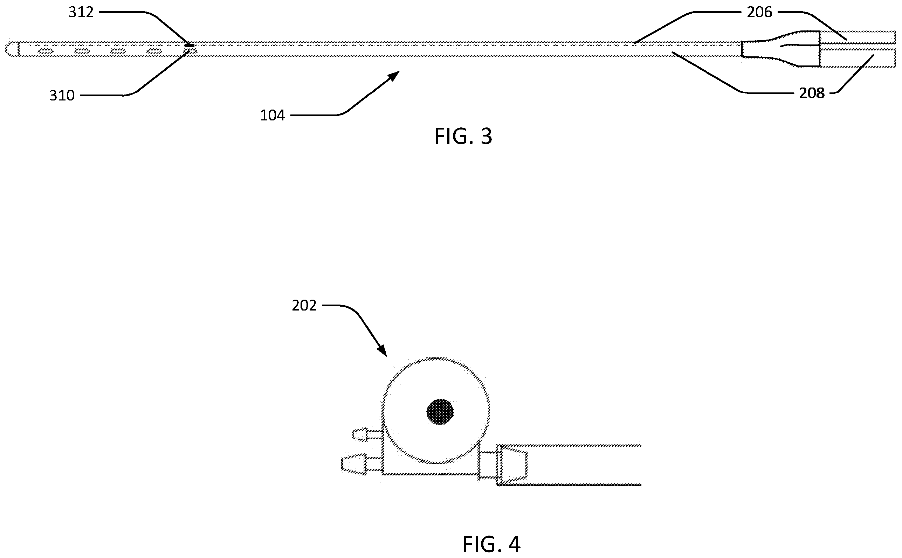

Fig. 3 shows an enlarged view of chest tube 104, including openings 310 for fluid drainage from the chest. Openings 310 are in fluid communication with chest tube drainage lumen 208. A radiopaque marker or markers may be incorporated into the chest tube. For example, a marker, such as marker 312, may be included at the most proximal drainage hole in the chest tube to act as a reference point for verification of depth of insertion and location of the chest tube within the patient. A marker may also be included at or near the distal end of the chest tube.

Fig. 4 shows an enlarge view of chest tube relief valve 202.

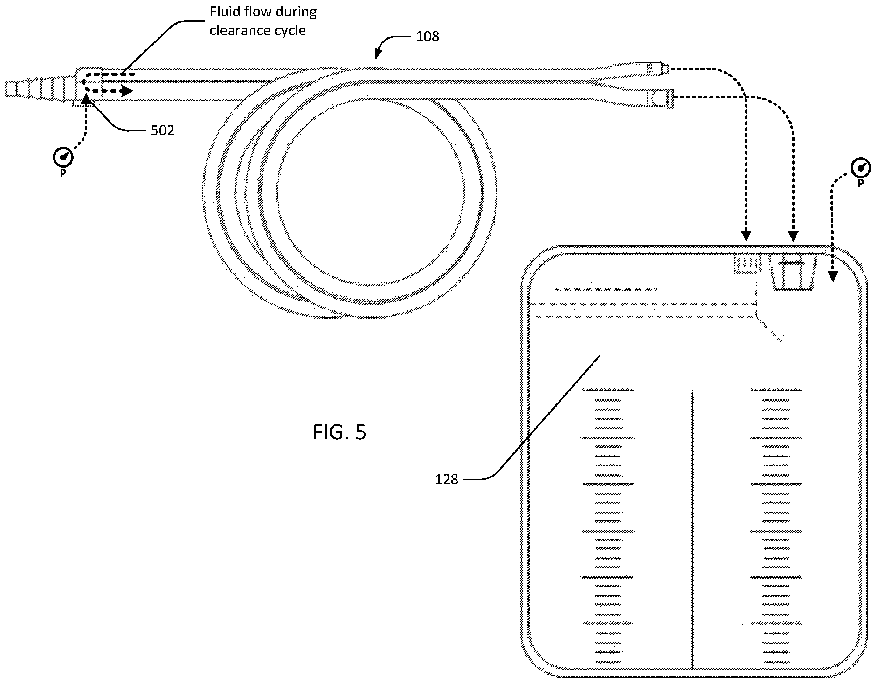

Fig. 5 shows an enlarged view of drainage tube 108 and canister 128. Preferably, the prescribed suction level is identical to or close to the suction level seen by the patient. The controller monitors pressure at the patient (at the drainage tube barb 502) using pressure sensor 116 connected pneumatically to the drainage tube barb via the drainage tube relief lumen of the drainage tubing. This pressure may be intermittently or constantly compared to pressure level set via the controller. If the pressure veers out of an acceptable range, the pump turns on or increases to reestablish the desired (set) negative pressure level. Any overshoot (pressure going too negative) during adjustment may be corrected by the controller (or manually) opening a solenoid valve in fluid communication with the drainage tube (at barb 502 or elsewhere) which allows atmospheric air to enter the system through a filter membrane (for example, through a 0.2-micron membrane).
When a drainage line purge cycle is initiated, the pump will turn on or increase and the drainage line relief valve will open to allow atmospheric air to enter the drainage tube relief lumen after passing through a filter membrane (for example, a 0.2-micron filter membrane). This air is pulled to drainage barb 502 and swept down the drainage lumen of the drainage tubing, along with other fluids, to the drainage canister. The pressure within the system during this process is affected by the pump RPM and the smallest inner diameter of the drainage tube relief valve (for example, a solenoid valve) and other tubing or channels that make up the drainage relief lumen. The pump may operate at a specified rate to maintain suction within the system without ramping up to dangerous levels.

During this process, pressure is also monitored at canister 128 to reduce the frequency of solenoid valve activation due to its proximity to barb 502.

### System Diagnosis Using Pressure Readings

Additionally, the two pressure sensor readings (at barb 502 and at canister 128) may be used and/or compared and analyzed by the controller and used to diagnose various situations occurring within the system:
1. If the canister sensor is reading the prescribed suction level and the barb pressure is reading a lower suction level (a less negative pressure) than the prescribed suction level, the system will initiate a drainage line clearance cycle.
2. If the controller pulls additional (up to 100 cmH₂O) suction and the canister pressure sensor reading shows a more negative pressure while the barb pressure reading does not change, the system will alert for an obstruction in the drainage line.
3. If the controller initiates a drainage line clearance cycle by pulling additional (up to 100 cmH₂O) suction and the barb suction level reading increases (pressure level decreases) past a set threshold, the controller may determine that the chest tube relief valve did not open to clear the chest tube and therefore the system will alert for a clog in the chest tube drainage line.
4. If the controller is being triggered to correct the system suction level frequently (more frequently than a set frequency threshold), a drainage line clearance or chest tube clearance cycle may be performed to clear any fluid buildup in the line and/or chest tube.
5. If the controller is being triggered to correct the system suction level frequently, it may be indicative of an active air leak, in which case the current air leak rate will be displayed on the controller display screen and/or an alert may sound.

Pressure sensor(s) may reside at various locations in the system. A pressure sensor may be incorporated within the chest tube valve device near chest tube, and/or near the controller, in the receptacle, or within or near the tube-tube interface area. Pressure sensors may also be located in other places in the system, for example, near the chest. Pressure sensed at one or more location may be used to determine whether there is a change in pressure anywhere in the system, which may be used to identify drainage tube blockages and/or chest tube blockages. If an impediment is detected, an audible alarm may sound, and/or the controller may automatically clear the drainage tube and/or chest tube.

### Chest Tube Detail

Fig. 6A shows a diagram of an embodiment of chest tube 104. Chest tube 104 includes chest tube drainage lumen 208 and chest tube relief lumen 206 incorporated into the chest tube. Chest tube relief valve 202 and filter/vent 604 are also shown in fluid communication with chest tube relief lumen 206, which is in fluid communication with chest tube drainage lumen 208 via opening 612. Drainage openings 310 allow fluid from the chest cavity to enter the chest tube and drain through chest tube drainage lumen 208. Generally when in use, openings 210 and opening 612 are inside the patient.

During successful chest drainage, chest tube relief valve 202 is in the closed position. In this position, fluid draining from the chest generally does not enter chest tube relief lumen 206 because of the fluid column in the chest tube relief lumen. A smaller diameter chest tube relief lumen may help prevent fluid from entering the chest tube relief lumen. The pressure in chest tube relief lumen 206 is slightly negative during chest tube drainage due to the negative pressure exerted by the pump on the drainage line, the chest tube drainage lumen, and to some extent, the chest tube relief lumen. The chest tube may become blocked or restricted, because of blood clots etc.

When a chest tube clog clearance cycle is initiated, the pump generates additional suction above the set level (to a more negative pressure) to open the chest tube relief valve. As this negative pressure drops to a set valve opening pressure, chest tube relief valve 202 opens, allowing atmospheric (i.e., more positive pressure) air to enter the chest tube relief lumen of the chest tube by passing through filter membrane 604. The air is pulled to the distal tip of the chest tube, through opening 612, and into the chest tube drainage lumen, where the air and other fluids are swept through the chest tube drainage lumen towards the drainage canister. This clearing is shown in Fig. 6B.

The chest tube relief valve is normally closed, as shown in Fig. 6A. The chest tube relief valve opens when the suction level applied by the pump to the drainage lumen is great enough (negative enough P) to overcome the forces applied to open the chest tube relief valve, as shown in Fig. 6B. When this occurs, the chest tube relief valve opens, allowing air to enter the system. The chest tube relief valve remains open until the suction level drops to the valve closing pressure. This pressure may be less negative than the valve opening pressure.

Once the pressure in the chest tube relief lumen increases back to a set valve closing pressure, chest tube relief valve 202 closes and normal drainage continues. The chest tube relief valve opening pressure may be different than the chest tube relief valve closing pressure. For example, the chest tube relief valve opening pressure may be at a higher pressure than the chest tube relief valve closing pressure.

For example, the chest tube relief valve may open when the pressure differential across the valve is about -10 cmH2O, about -20 cmH2O, about -30 cmH2O, about -40 cmH2O, about -50 cmH2O or as even high as about -100 cmH2O. Or for example, the chest tube relief valve may open when the pressure differential across the valve is within a range of about -10 cmH2O to about -20 cmH2O, or within a range of about -20 cmH2O to about -30 cmH2O, or within a range of about -30 cmH2O to about -30 cmH2O, or within a range of about -40 cmH2O to about -40 cmH2O, or within a range of about -50 cmH2O to about -100 cmH2O.

The chest tube relief valve may close at the same range, or at a lower differential than the opening pressure. For example, the chest tube relief valve may close at a pressure differential of about to 0 cmH2O, about -5 cmH2O, about -10 cmH2O, about -15 cmH2O, or about -20 cmH2O. Or for example, the chest tube relief valve may close at a pressure differential range of about to 0 cmH2O to about -5 cmH2O, or a range of about -5 cmH2O to about -10 cmH2O, or a range of about -10 cmH2O to about -15 cmH2O, or a range of about - 15 cmH2O to about -20 cmH2O.

The chest tube relief valve may take a variety of known forms, including but not limited to a check valve, umbrella valve, ball valve, Belleville valve, X-fragm valve, cross-slit valve, or dome valve. The valve system preferably has a filter in place to prevent the entrance of bacteria or viruses from the atmosphere into the patient.

In another embodiment of the chest tube, chest tube relief valve is active, not passive, and is controlled directly by the controller. In some embodiments of the chest tube, chest tube relief valve is operated manually.

In some embodiments of the chest tube, chest tube relief valve is incorporated into the chest tube. In some embodiments, the chest tube relief valve is incorporated into a connecter which is connected to the chest tube. In some embodiments of the chest tube, both the chest tube relief lumen and the chest tube relief valve are incorporated into a connecter which may be connected to a chest tube.

In some embodiments, chest tube relief valve 202 takes the form of a magnetic check valve that has a substantial difference in the pressure differential required to open the valve, and the pressure differential required to keep the valve open (or close the valve), thereby amplifying the toggling effect of the valve. This is preferable to increase the effectiveness of the clog clearance cycle, because it allows for a greater pressure differential when the air is sweeping the chest tube drainage lumen via the chest tube relief lumen than if the valve opened and closed at the same pressure. The valve is normally closed in order to maximize drainage of liquid as it enters the chest tube and to reduce the need for continuous pumping.

### Chest Tube Relief Valve

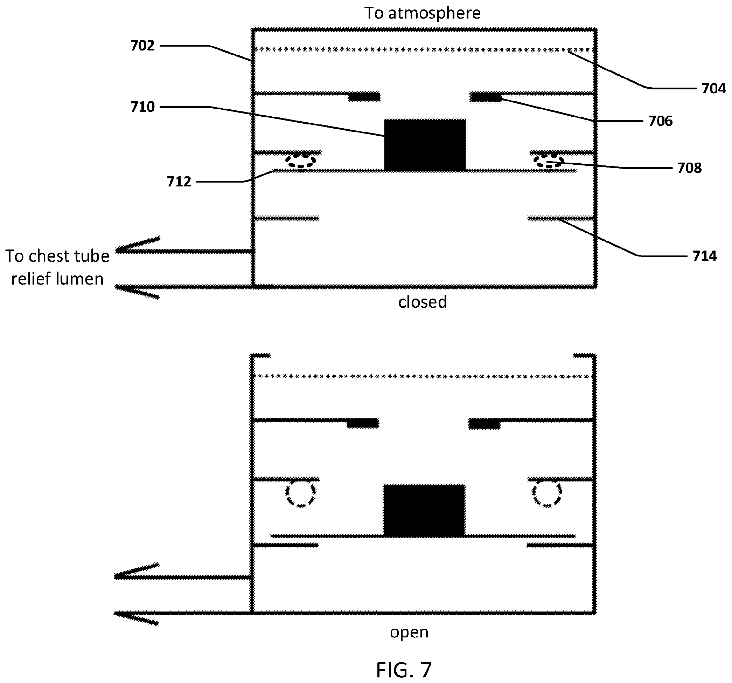

Fig. 7 shows a magnetic embodiment of the chest tube relief valve. The magnetic chest tube valve includes housing 702, filter 704, ferrous plate 706, gasket 708, magnet 710, seal plate 712, and positioning lip 714. When the pressure differential across the valve increases above a desired threshold, for example -50 cmH2O, the force caused by the pressure differential is enough to overcome the magnetic force between the magnet and the ferrous plate, thereby moving the two away from each other. Once the magnet and the ferrous plate move away from each other, the magnetic force rapidly diminishes, as the magnetic force is proportional to (1 / r³) where r is the distance between the magnet and the plate. At the same time, the opposing spring force also diminishes, but less rapidly, as it is proportional to (x), where x is the length of spring that has been compressed. Therefore, once the seal plate moves slightly away from the gasket, the spring force can overcome the magnetic force and push the seal plate into the completely open position (the pressure during this time remains relatively constant due to the reservoir of suction in the system). As a result, the amount of pressure necessary to keep the valve open is less than the pressure that was required to open it. This second pressure value, for example -50 cmH2O, is determined by the maximum distance the magnet and seal plate can travel away from the ferrous plate, which is in the embodiment shown in Fig. 7 determined by positioning lip 714 in the housing that sets this distance.

Frequency of clog clearance function activation at the controller may be 5 minutes or may be longer such as every 10 or 15 minutes. Air leak measurements may be temporarily suspended during the clog clearance cycle. The clog clearance cycle (either chest tube clearing or drainage tube clearing) frequency may be set by the user via the display/input interface.

Another embodiment of the chest tube relief valve may allow for selectable crack pressures. The activation mechanism may take the form of a button, switch, dial, or similar implement that mechanically alters the crack pressure by adjusting the stand-off distance of the magnetic seal allowing either higher or lower transmitted suction levels to open the valve. The adjustment can be a permanent alteration to the activation level of the relief valve or as a temporary override. A similar outcome may be achieved by altering the electromagnetic field in the proximity of the valve.

In yet another embodiment of the chest tube relief valve the clinician may temporarily open the chest tube relief valve allowing for the inflow of air to the chest tube relief lumen. This can either open the internal valve of the chest tube relief valve or bypass the chest tube relief valve by opening a separate bypass port in communication with the chest tube relief lumen. The activation mechanism may take the form of a button, switch, dial, or similar. The function can be used to assist in the activation of, or be used in place of, a chest tube clog clearance cycle. For example, the temporary or bypass function may be used to initiate clog clearance on a more frequent interval than that of the controller, to ensure clear chest tubes and drainage lines prior to measurement of fluid output, in place of the automated clog clearance feature, or for use with a different type of suction system. The bypass or adjustment can be a permanent alteration to the activation level of the relief valve or as a temporary override.

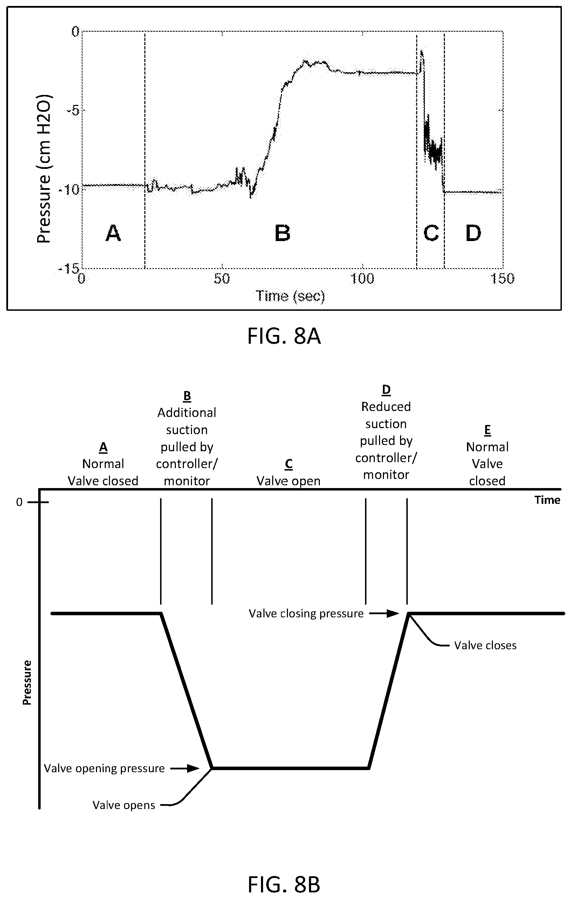

Fig. 8A shows the chest drainage system's ability to detect and clear pooled liquid in the drainage tube. In section 'A', a -10 cmH2O vacuum is properly transmitted to the chest. In section 'B', liquid begins to pool in the drainage tube, resulting in a decreased negative pressure (or an increased pressure) as sensed at or near the tube-tube interface area. If unresolved clinically, drainage would be impeded. However, in section 'C' the drainage tube relief valve is opened and the liquid is flushed into the drainage container, resulting is restoration of proper suction in Section 'D', as well as proper negative pressure as measured. The valve is closed after normal drainage/pressures have been restored. In this example, the pressure is measured at the tube-tube interface area, however pressure may be measured in other and/or additional locations in the system. For example, pressure may be measured at or near the chest or chest tube and also at or near the suction device and/or canister. In some embodiments, the differential pressure measurement may be used to detect flow impediments or pooling or clotting of blood/fluid. For example, in embodiments where the pressure is measured in the collection canister, the measured pressure would get more negative in the presence of a blocked drainage tube.

The controller can identify impediments to fluid drainage via a measured absolute pressure, change in pressure, pressure differential between or among 2 or more locations, or at one location. When an impediment to fluid drainage is identified, an alarm may sound and/or the controller may initiate clearing procedures, including opening and/or closing valve(s) in the chest drainage system, as described elsewhere herein. The negative pressure in the drainage tube may be increased, or changed in other ways, such as pulsed, reversed etc.

For example, if pressure measured at the tube-tube interface area is reading around -10 cmH20 to around -20 cmH20 and the reading changes to zero to -5 cmH20, the controller may open the drainage tube valve to filtered atmospheric air. The controller may leave the valve in this open position for a set period of time, for example, 5-10 seconds or 10-30 seconds and then may return the valve to its regular closed position. Alternatively, the controller may close the valve when a set pressure is measured at the tube-tube interface area or elsewhere. The controller may then check the pressure readings and if they have returned to normal, do nothing more. If they have not returned to normal, indicating a blockage or slowing condition is still present, the controller may repeat the clearing procedure. This may be done repeatedly until the tubing is cleared. Alternatively or additionally, the procedure may change if repeat clearings are necessary. For example, the magnitude of negative pressure used by the suction device to clear the tubing may be increased, and/or the negative pressure may be pulsed. The clearing procedure may be performed in response to the pressure readings and/or it may be done automatically on a periodic basis.

Figs. 8B-8F shows the chest drainage system's ability to detect a blocked chest tube. Fig. 8B shows the pressure in the chest drainage system over time, as measured at the tube-tube interface. This pressure may be measured by the controller, preferably via the drainage tube relief lumen, but can alternatively be measured elsewhere.

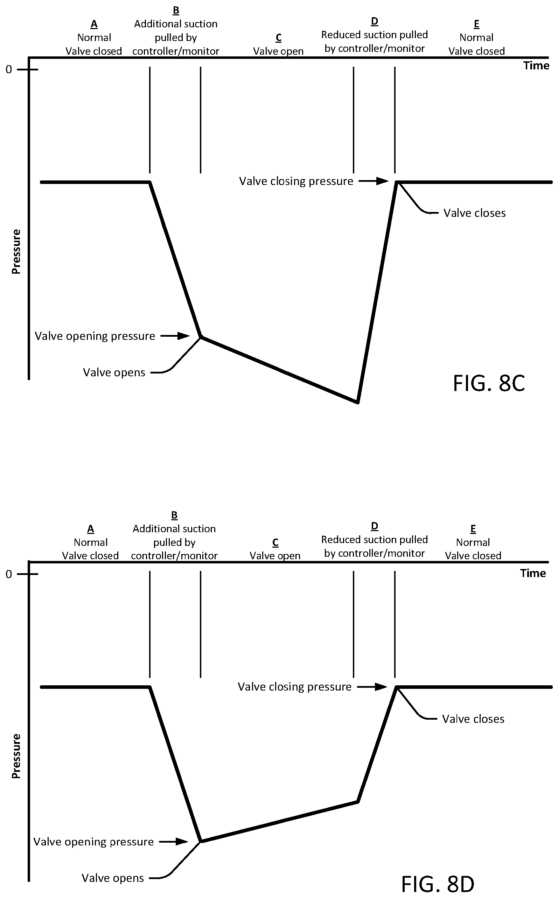

Section A of Fig. 8B shows normal drainage using at a negative pressure created by the suction pump. Section B shows additional suction being pulled by the controller/monitor. This additional suction may be pulled periodically, or may be pulled based on pressure readings in the system. For example, additional suction may be pulled when the presence of tidal oscillations is no longer detected in the drainage system by the controller. The additional suction transfers negative pressure to the drainage tube drainage lumen, the chest tube drainage lumen, and ultimately the chest tube relief lumen and chest tube relief lumen valve. When the pressure differential across the chest tube relief lumen valve reaches the valve opening pressure, the chest tube relief lumen valve opens. The valve may open automatically if the valve is passive, or directly by the controller, if the valve is active. In some embodiments, the valve may be opened manually. Section C shows the pressure when the valve is open. The valve may remain open for a set period of time. Alternatively, the valve may remain open until the controller senses that the clog has been cleared. The negative pressure, or suction, within the system may remain steady during this phase, as shown in Fig. 8B, or the negative pressure may become more negative, as shown in Fig. 8C, or the pressure may become less negative, as shown in Fig. 8D.

Section D shows the magnitude of the negative pressure decreasing as a result of a reduction in suction being pulled by the controller/monitor. When the pressure in the system reaches the valve's set closing pressure, the valve closes (or is closed) and fluid drainage continues in a normal manner. The valve closing pressure may be at a lower magnitude negative pressure than that of the opening pressure, as shown here. The valve closing pressure may be at or near normal drainage negative pressure.

Figs. 8B-8D show different slopes of negative pressures in different situations. In Fig. 8B the rate at which air is entering the system via the chest tube relief lumen valve is the same as the rate at which the suction pump is draining the system during the open valve section C. In Fig. 8C, the rate of drainage is higher than the rate of air entering the system. In Fig. 8D, the rate of drainage is lower than the rate of air entering the system. The slope of the pressure curve in section C may be controlled by the controller and the amount of suction that it is pulling. The slope may also be reflective of whether a clogged chest tube is being cleared or not being cleared, as is described elsewhere herein.

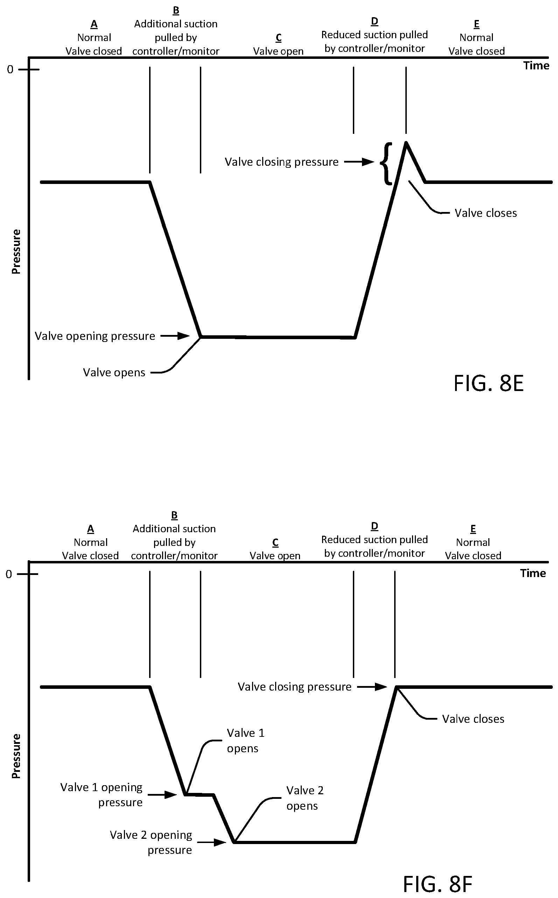

Fig. 8E shows an embodiment where the controller "overshoots" the normal draining suction pressure to close the chest tube relief lumen valve. The valve closing pressure in this embodiment may be around the normal draining pressure, or it may be at a less negative pressure (lower differential pressure).

Fig. 8F shows an embodiment where there is more than one chest tube. In this embodiment, the first chest tube relief valve opens when the pressure in the system reaches valve 1 opening pressure. It may be necessary to increase the magnitude of the negative pressure in the system further to open the second chest tube relief lumen valve. This is shown as valve 2 opening pressure on the graph. There may be 1, 2, or more valve opening pressures depending on how many chest tubes are used on a single patient. The closing pressures of the multiple chest tube relief valves may be the same, or they may be different. The ability to detect the opening of the valves may be useful to determine whether one or more of the chest tubes is clogged, in which case an alarm or notification may be provided, or the clog may be actively cleared by the system.

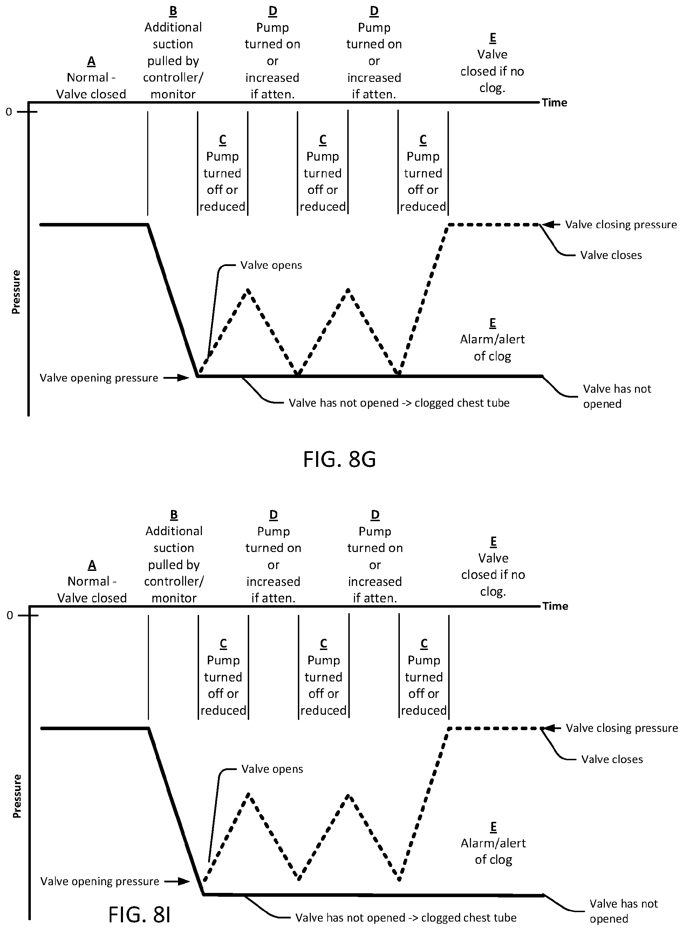

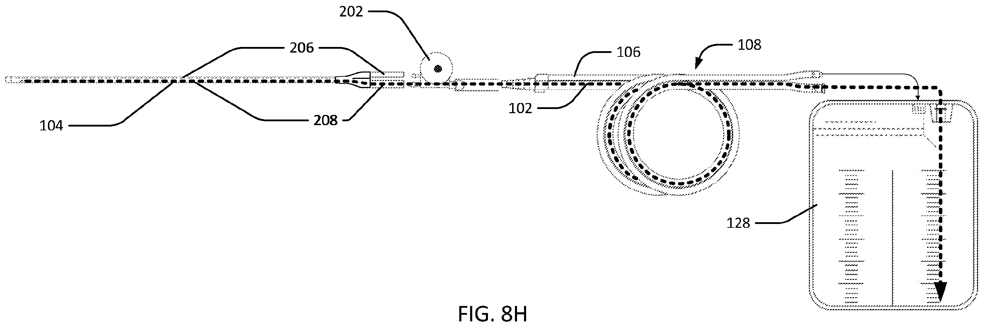

Fig. 8G shows the chest drainage systems ability to detect chest tube clogs using pressure readings. Pressure readings may be taken at or near the tube-tube interface, or at or around the drainage canister. Pressure readings for this purpose are preferable downstream from the chest, so in the downstream portion of the chest drainage system (shown as a heavy dashed line in Fig. 8H). Section A shows the controller pulling normal drainage level of suction, and the chest tube relief valve is closed. Section B shows additional suction being pulled (more negative pressure being applied) via the canister. The measured pressure becomes more negative. When the suction is near, or above, the chest tube relief valve opening pressure (the pressure differential across the valve is at or near the crack pressure of the valve), the valve may open, as shown by the dotted line, and the suction may then be reduced as air passes through the chest tube relief lumen, into the chest tube drainage lumen, and through the drainage tube drainage lumen. The dotted line represents a chest tube which is not clogged, or which was clogged, but has been cleared via the increased suction and the opening of the chest tube relief valve. Section C represents the turning off, or reducing, or the suction applied to the chest tube drainage lumen (via the drainage tube drainage lumen).

However, if there is a significant clog in the chest tube, the chest tube relief valve may not open, even with the increased suction. This case is represented by the solid line. The controller of the chest drainage system may increase and reduce the suction multiple times during the clog detection process. Section D shows the pump increasing the suction if suction attenuation has been sensed (i.e., an increase in pressure, or a reduction in suction in the canister or at the tube-tube interface)

If no chest tube clog is present, or if the increased suction level has cleared the chest tube, the suction will attenuate back down to the valve closing pressure after the suction is reduced. This is shown in section E. Alternatively, if a chest tube clog remains, the measured suction (negative pressure) will remain relatively un-attenuated, as shown by the solid line, and the controller may trigger an alarm or alert to notify the user that a chest tube clog is still present.

Fig. 8I shows pressure readings measured when the chest tube clog detection cycle causes the controller to increase the section level beyond (more negative than) the chest tube relief valve opening pressure.

In some embodiments of the chest drainage system, the chest tube and/or drainage tubing clog detection techniques described herein may be used to confirm the patency of the chest tube prior to removing the tube from the patient. This may be particularly valuable, for example, when the air leak value has diminished to zero or near zero for a prolonged period of time, at which point the system may either automatically or manually activate a drainage tubing and/or chest tube clog clearance cycle in order to confirm patency of the chest tube. In doing so, the system provides confirmation that the cessation of air leak is due to actual physiological healing and not because the tube itself has become obstructed.

In some embodiments, the chest drainage system may include a pH sensor. Post-surgery infection and empyema are of particular concern to clinicians. The pH of fluid drained from the body can be useful in diagnosing these, and other, conditions. To aid in the diagnosis, the chest drainage system may include a pH monitor in the controller, with a sensor in the reservoir, in the tubing, the pump, the valve device, or anywhere in the system. The results may be displayed on the display device. The system may also include a sampling port to sample the fluid drained from the chest. The system may also include an infusion port to infuse an additive into the drainage fluid. These ports may be in the reservoir, tubing, controller, valve device, or elsewhere in the system, for example at the tube-tube interface.

In some embodiments, pH of the drained fluid is measured to monitor for infections. Additional parameters, such as conductance, spectroscopic signatures, protein content, and specific gravity of the drained fluid may also be measured to monitor patient recovery. Any of these measurements may be one time measurements or measurements made over time. For measurements made and collected over time, the controller may analyze these data for trends. These data may be integrated with the hospital's electronic medical record system (either communicated to, or data may be obtained from) and/or displayed on a screen on the device or on a connected monitor, which may be connected either by wire or wirelessly. In some embodiments, alarms or notifications may be activated by the controller when the parameters surpass certain thresholds, which may be preset or set by the user. These may be visual and/or audible alarms or notifications. These data may also provide input to the line-purging and clog-clearing functions of the device, such that, for example, line purging is activated when the suction at the chest drops below a certain level, or clog clearing is activated when tidal oscillations are diminished.

### Air Leak

In some embodiments, the system is capable of measuring the flow rate of air evacuated from the canister/reservoir, in addition to pressure in the canister and pressure in the drainage tube relief lumen. Evacuation flow rate may be used to determine the presence and rate of an air leak from the chest cavity. The evacuation flow rate necessary to maintain the system at the prescribed suction level is equivalent to the flow rate of air entering the system (air leak), as the flows of air into and out of the system must be equal in the presence of steady pressure. Evacuation flow rate may be determined by the flow rate of the air being evacuated from the canister via the integrated suction pump and the volume of liquid in the canister. These parameters may be tracked over time by the controller to determine chest air leak presence and other parameters, such as air leak rate and changes to the air leak rate over time. Flow rate measurements can be made with any number of off-the-shelf sensitive air flow sensors that are known in the art. Flow rate may alternatively or additionally be measured by measuring the revolutions of the pump motor necessary to keep the suction at a prescribed level via a tachometer.

To determine the air leak rate within the system, the may utilize the pump tachometer to count the rotations that the pump is undertaking in creating the suction within the system. The rotation number, as well as pressure measurements and time measurements may be used to identify and quantify air leaks. The quantification of air leak rate to milliliters per minute may be achieved by the controller by using a transfer function to convert pump revolutions per minute (RPM), and canister pressure, to milliliters per "tick" of the pump rotation counter. This transfer function may depend on the suction level of the system and the speed of the pump. The number of "ticks" may then be multiplied by the milliliters per "tick" metric (based on the current RPM and pressure) and divided by the time over which the "ticks" occurred, resulting in an average air leak rate in milliliters per minute.

In some embodiments, the volume and/or flow rate of an air leak (from the patient's lung) is measured to monitor wound healing.

### Drained Fluid Volume Measurement

Collected fluid (either gas, liquid, or both) volume and/or flow rate measurements may be made over time, and the data stored and/or displayed and/or shared with other systems. The volume measurements may be made with a non-contact capacitive sensor, but may alternatively be made with optical sensors, pressure sensors, acoustic (such as ultrasonic) sensors, or any other liquid level sensing methods known in the art.

To determine the drainage fluid volume collected in the drainage canister, the controller may utilize a built-in capacitive level sensor that senses changes in capacitance between two electrodes as the height of fluid in the drainage canister changes. The controller may utilize a transfer function to convert capacitance and directional tilt (measured by accelerometers, gyroscope, camera, etc.) of the controller/monitor into fluid volume in milliliters or other appropriate units.

In some embodiments, a capacitive sensor is mounted on the inside of the controller and may use out-of-phase techniques to reduce interference from within the proximity, such as a human hand near or in contact with the container. Such a technique uses a level electrode, reference electrode, environment electrode, ground electrode, and two shield electrodes. In some embodiments, the drainage volume is calculated by dividing the change in capacitance of the level electrode by the change in capacitance of the reference electrode and multiplying by the known volume that corresponds to the height of the reference electrode. In this embodiment, the height of reference electrode is a fraction of the height of the level electrode, for example but not limited to 1/10^{th}, 1/20^{th} or 1/50^{th} of the height of the level electrode. In another embodiment, a compliant layer of material is present on either the controller or the suction canister in the area of the capacitive electrode in order to minimize or eliminate any air gaps between the controller and the suction canister.

Drainage fluid volume may be measured and tracked in the presence or absence of air leak determination.

In some embodiments, the controller is connected to a network, either wired or wireless, in order to transmit data for example to and/or from the patient's electronic medical record (EMR). The controller may also provide notifications of patient status on the controller/monitor itself and/or by transmitting notifications and/or safety alarms to the EMR or the clinician's phone, tablet, watch, etc.

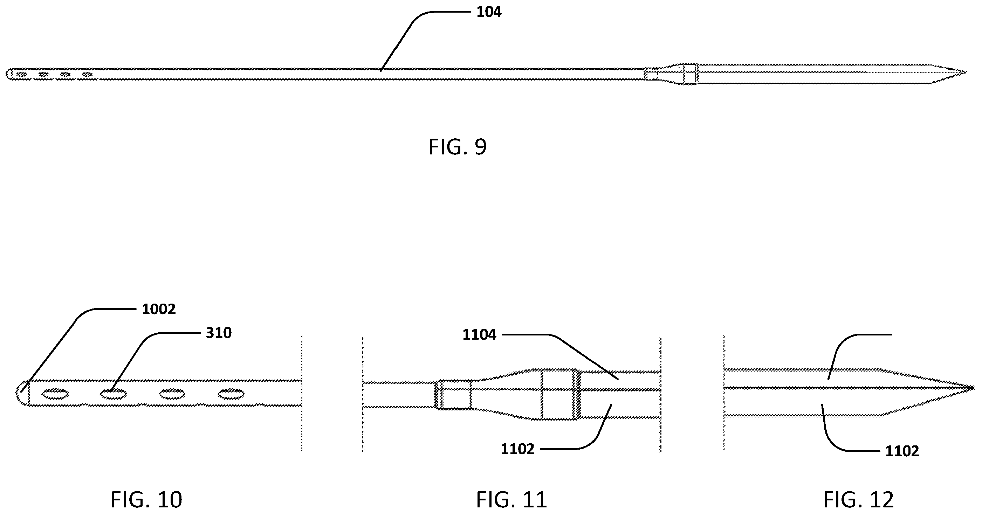

Figs. 9, 10, 11 and 12 show an embodiment of a dual-lumen chest tube. Chest tube 104 may be made using silicone, PVC, or other suitable material with a suitable durometer, for example 20A - 80A. The effective outer diameter of the chest tube may vary between 8Fr - 40Fr. For example, chest tube sizes may include 15 Fr., 19 Fr., 23 Fr., 27 Fr., etc. The chest tube shown in Fig. 9 may include three sections: a chest tube region, as shown in Fig. 10, a transition region, as shown in Fig. 11, and a pull-through region, as shown in Fig. 12. The chest tube region comprises a dual-lumen extrusion with holes 310 near the patient side for drainage of fluid from the body. The chest tube region is preferably capped with rounded tip 1002, but may also have an open patient end without a cap. The transition region separates the two chest tube lumens, for example chest tube drainage lumen and the chest tube relief lumen, into separate tube sections that are more easily attached to barbed connectors.

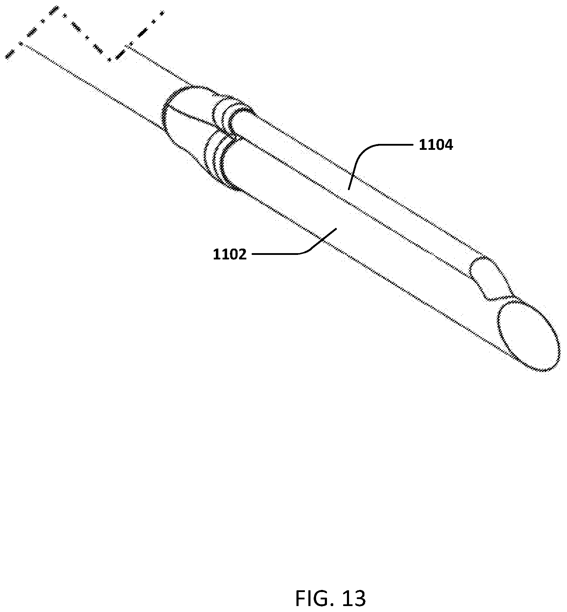

Fig. 11 shows chest tube drainage lumen tube section 1102 and chest tube relief lumen tube section 1104. Specifically, at the non-patient end of the transition region, both lumen preferably become circular to allow for proper attachment to standard barbs. The pull-through region shown in Fig. 12 includes chest tube drainage lumen tube section 1102 and chest tube relief lumen tube section 1104. The two tube sections may also be joined, for example with webbing or adhesive. The ends of the two tubes may be tapered to allow for easier insertion into the chest and also easier pulling of the chest tube through, from the inside to the outside, the chest wall. Alternatively, the tubes may not be tapered or only one of the tubes may be tapered. In some embodiments, the relief lumen tube may "dive" into the larger tube so the outer profile on the non-patient end is just that of the drainage tube. This is shown in Fig. 13. The relief tube is also preferably sealed near the non-patient end, for example with a plug of silicone, in order to prevent fluid ingress into the relief lumen as the tube is pulled through the patient wall.

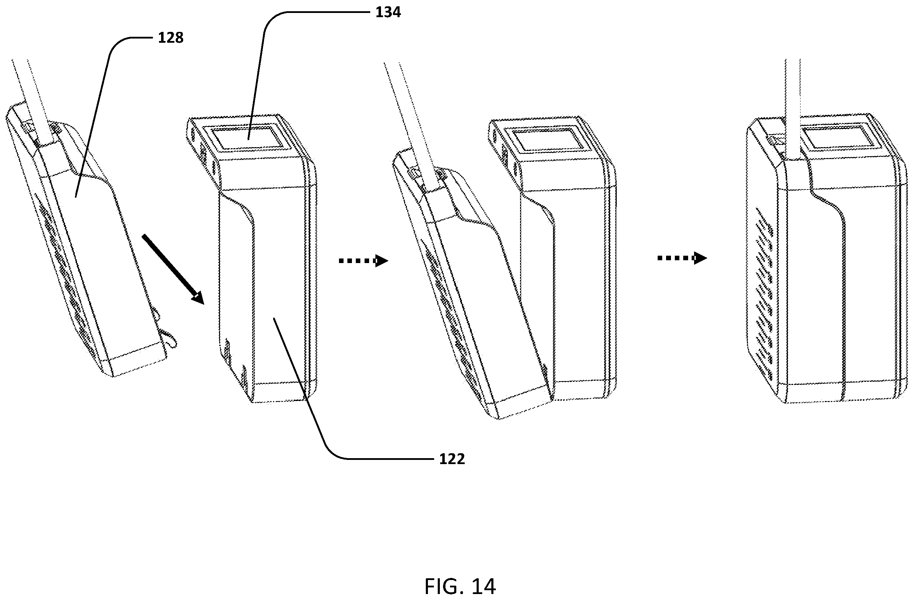

In some embodiments, the chest drainage system includes the monitor/controller shown in Fig. 14. In one embodiment, the monitor includes screen 134, integrated pump (not shown) and mating ports between suction canister/reservoir 128 and monitor 122, including ports to provide suction to the reservoir, open the drainage tube relief lumen valve via integrated solenoid or other means, and capture/secure the drainage tubing and suction canister. In some embodiments, the pneumatic lines are protected by filters integrated into the canister itself to prevent egress of liquid from the canister.

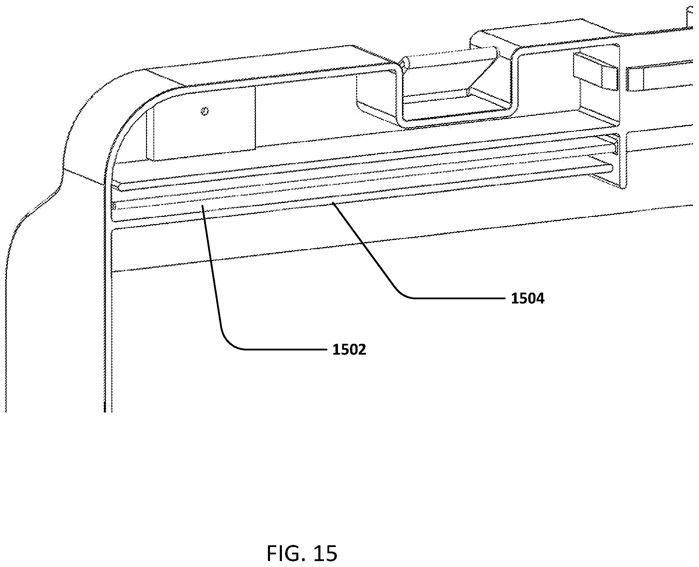

In some embodiments, the suction canister/reservoir is protected from liquid egress by means of a tortuous path created by the internal geometry of the suction canister/reservoir as shown in Fig. 15. The tortuous path may include a series of ribs 1504 and channels 1502 to separate the fluid collection chamber of the reservoir from the vacuum/suction port which connects to the monitor. The tortuous path geometry makes it more difficult for liquid to reach the suction port regardless of monitor orientation. The series of ribs may be connected to the drainage canister on one end and just short of reconnecting at the other end, creating a slight gap. These physical barriers are effective when the drainage canister is laid on its front, back, left, or right sides.

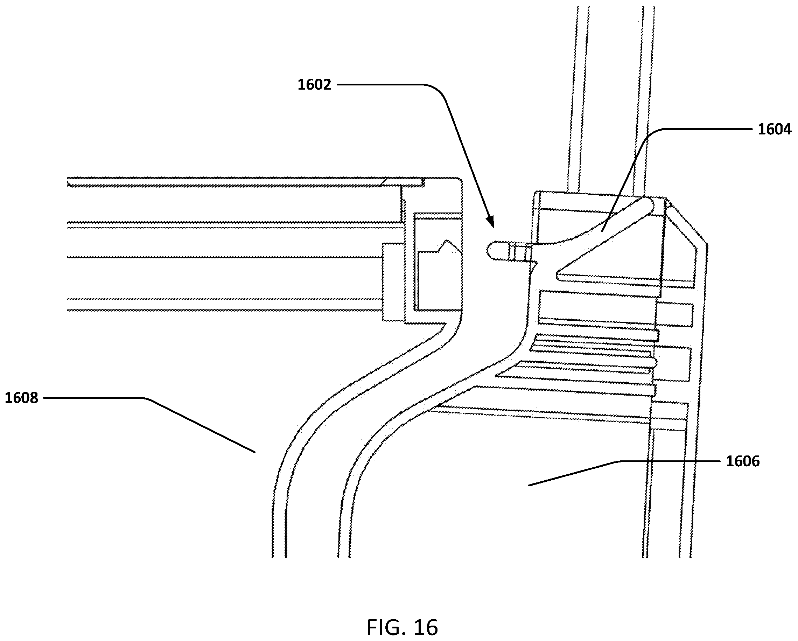

In some embodiments, an accelerometer is used to monitor orientation of the monitor and the controller provides an alert when the monitor is in a position that may compromise the suction port. In this example embodiment, the drainage tubing is first connected to the drainage canister and the drainage canister is then connected to the monitor. Alternatively, the drainage tubing drainage lumen and/or drainage tube relief lumen may be connected to the monitor itself, and/or the two tubes (drainage tube drainage lumen and drainage tube relief lumen) may be connected separately. In the embodiment shown, the canister/reservoir is connected to the front of the monitor, but in other embodiments may be connected to the back or either side of the monitor, or be separate. In one embodiment, the suction canister/reservoir has a latching hinge that mates with a latch on the controller as shown in Fig. 16, such that once the canister is connected to monitor 1608, hinge 1604 must be manually depressed in order to disengage latch 1602 and remove canister 1606 from monitor 1608.

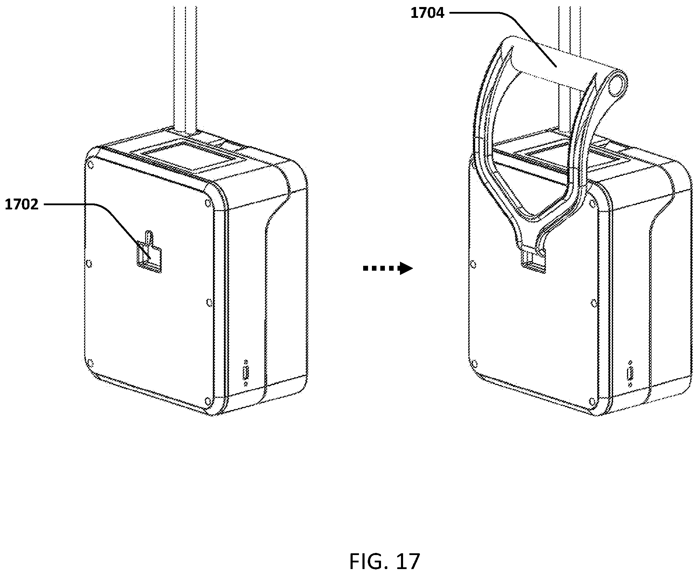

In another embodiment of the device shown in Fig. 17, the monitor has modular attachment receptacle 1702 for accepting any number of accessories for mounting or handling the device, including but not limited to bed mounts, IV pole mounts, carrying straps, or handle 1704, as shown in Fig. 17. In another embodiment, the device may have multiple such attachment receptacles to allow for multiple accessories to be connected at once, for example but not limited to a bed mount and a handle or a handle and carrying straps.

### Multiple Chest Tubes

Embodiments of the chest drainage system may include the ability to support more than one chest tube. For example, the system may support up to 2 chest tubes. Alternatively, the system may support up to 3 chest tubes. Alternatively, the system may support up to 4 chest tubes. Alternatively, the system may support up to 5 chest tubes. Alternatively, the system may support up to 10 chest tubes. Some embodiments include the ability to configure the system to be used with one or more "off the shelf" chest tubes. "Off the shelf" (OTS) chest tubes may not include a chest tube relief lumen or a chest tube relief valve. For example, in a system which supports 3 chest tubes simultaneously, the controller may be configured to support any combination of OTS chest tubes and proprietary chest tubes (chest tubes with a chest tube relief lumen and a chest tube relief valve) (P). For example, a 3 chest tube system may be configured for:
P P P
P P OTS
P OTS OTS
OTS OTS OTS

The system may alternatively be configured to be used with fewer than the maximum number of chest tubes it supports.

The controller may pull additional suction sufficient for activating/opening multiple chest tube relief valves simultaneously, or in succession, for example as shown in Fig. 8F. In configurations where an OTS chest tube is used with embodiments herein which include a chest tube relief lumen and chest tube relief valve, the controller may only need to supply enough suction to open the chest tube relief lumen valves. In other words, for example, the graph shown in Fig. 8F may apply to any combination of chest tubes which only includes two chest tube relief valves, such as:
P OTS OTS
OTS OTS
P P OTS OTS

Note that in configurations where the chest drainage system is used with OTS chest tubes without a chest tube relief lumen/valve, the drainage lines connected to the OTS chest tubes may still be cleared using devices and methods disclosed herein. In other words, an OTS chest tube may be connected to a drainage line which has a drainage line relief lumen and drainage line relief valve.

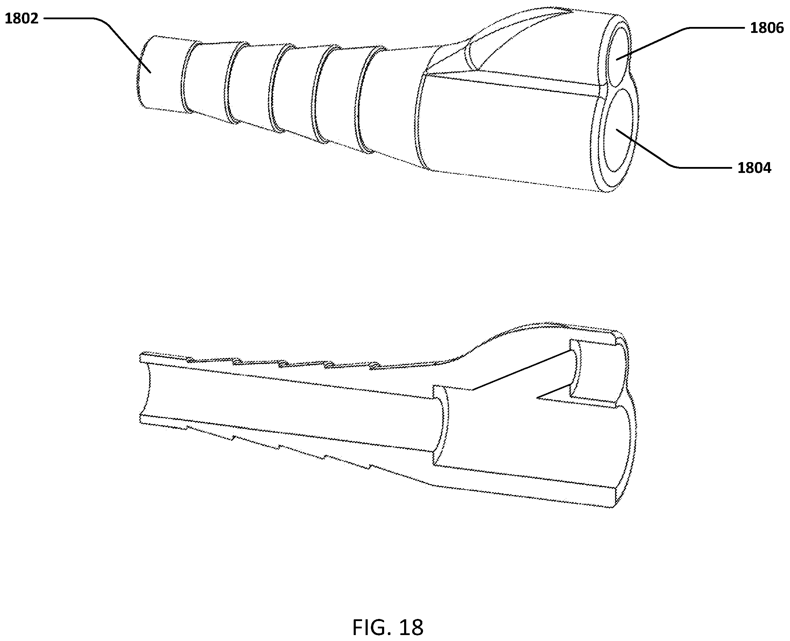

In embodiments where the chest drainage system is used with a standard OTS chest tube without a chest tube relief lumen, the drainage tube relief lumen and drainage tube lumen may join together at a connection barb between the drainage tube and the chest tube. An example of this type of connection barb is shown in Fig. 18. The connecter includes chest tube connecter 1802, drainage lumen connecter 1804 and drainage lumen relief lumen connecter 1806. This connecter arrangement may be particularly appropriate in thoracic surgery where there is less concern of clogging within the chest tube, and clearance of the drainage line to maintain suction pressure is the primary concern. In another embodiment, the same type of connection barb may be used with a chest tube with a chest tube relief lumen that includes any of the chest tube relief lumen passive valves described herein. In this configuration, the passive valves are normally closed, but the pump in the monitor may generate additional suction at temporal intervals (or when a blockage is sensed) in order to surpass the crack pressure of the valve such that it opens and air can sweep the chest tube drainage lumen clear via air from the chest tube relief lumen. This activation may alternatively or additionally occur when the monitor detects that the magnitude of tidal oscillations has diminished, indicating that a blockage is forming within the chest tube. The controller may also temporarily reduce the suction magnitude after such an activation is performed in order to ensure that the passive valve closes again.

### User Interface

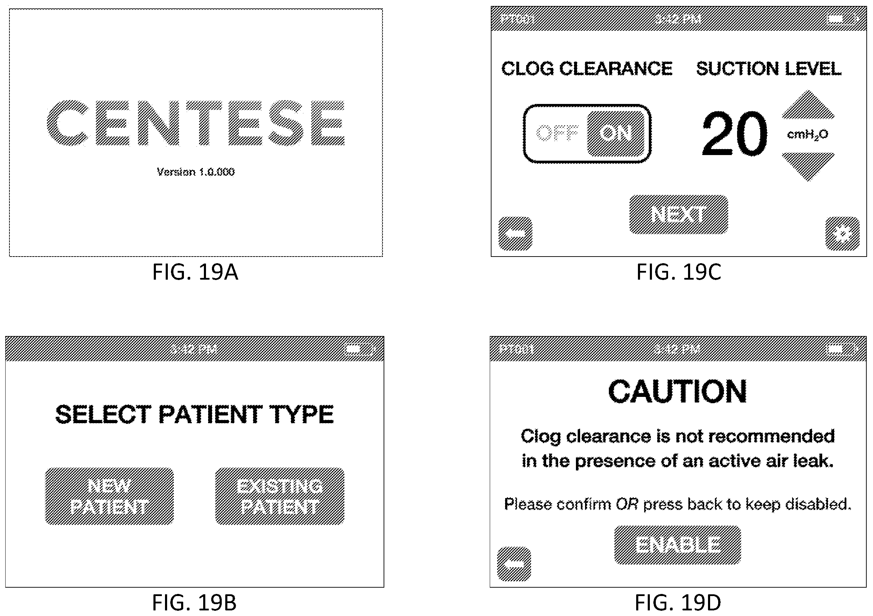

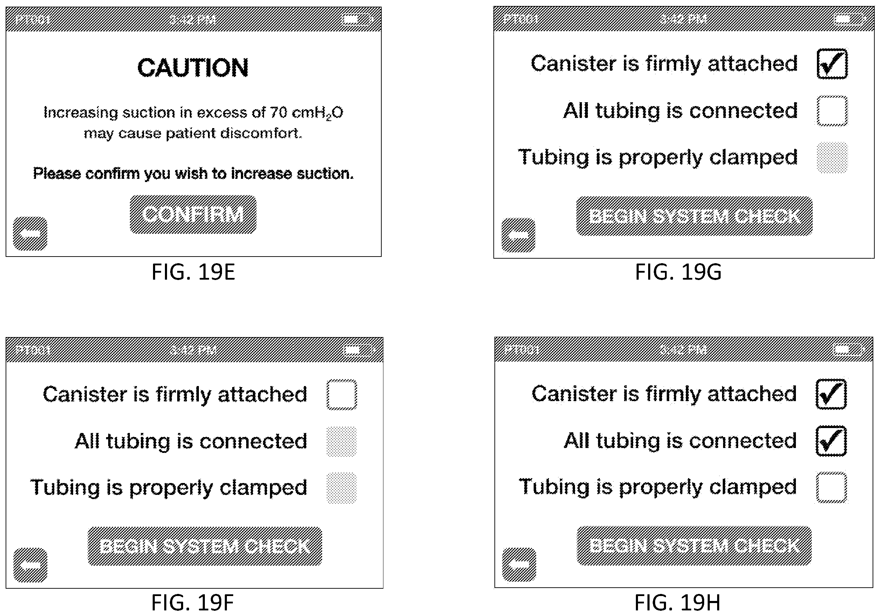

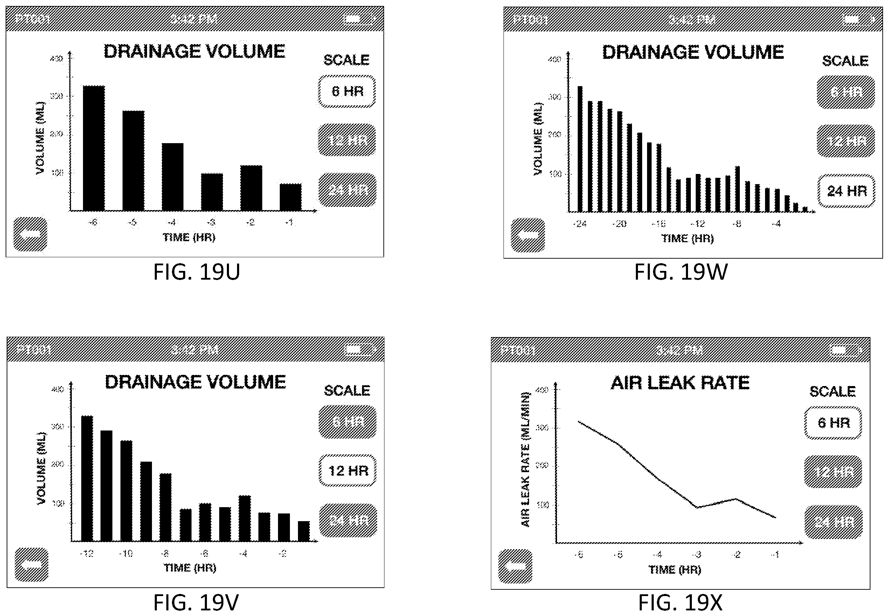

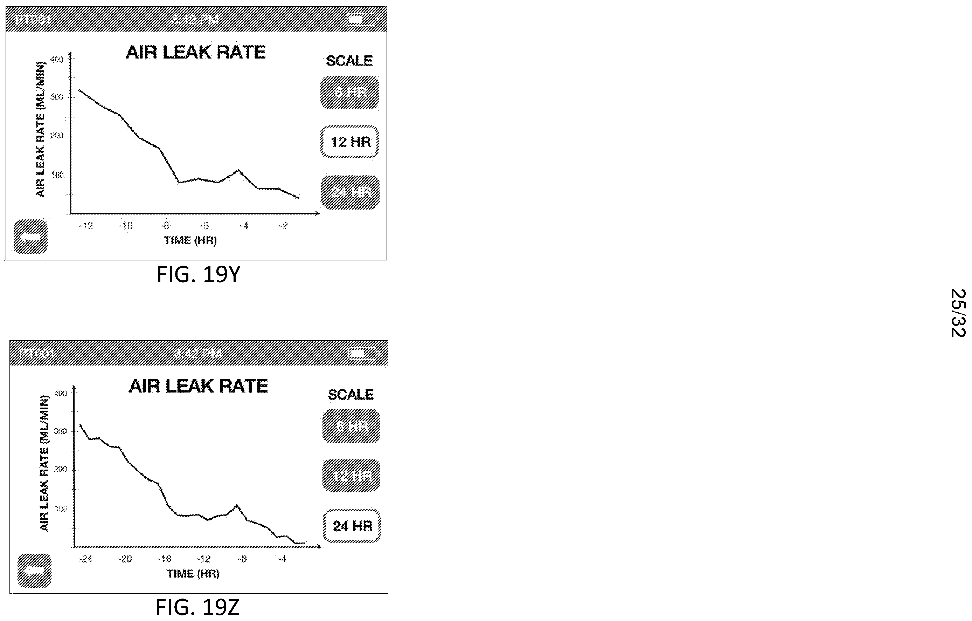

In some embodiments, a graphical user interface is displayed on the display of the controller. In some embodiments, the Graphical User Interface (GUI) may include the screens depicted in Figs. 19A-Z. These screens may display information, as well as collect input from the user, for example via touch screen input. Upon boot up of the device, a flash screen is displayed, which includes the current version of the software (Fig. 19A). The user is then prompted to select whether the patient is new or existing (Fig. 19B). Next, the user sets the clog clearance feature to on or off and also sets the initial suction level for the patient (Fig. 19C). If clog clearance feature is turned on, the user may be prompted to confirm (Fig. 19D). Similarly, if the user attempts to increase the suction level to higher than 70 cmH2O, he/she may be asked to confirm the setting (Fig. 19E).

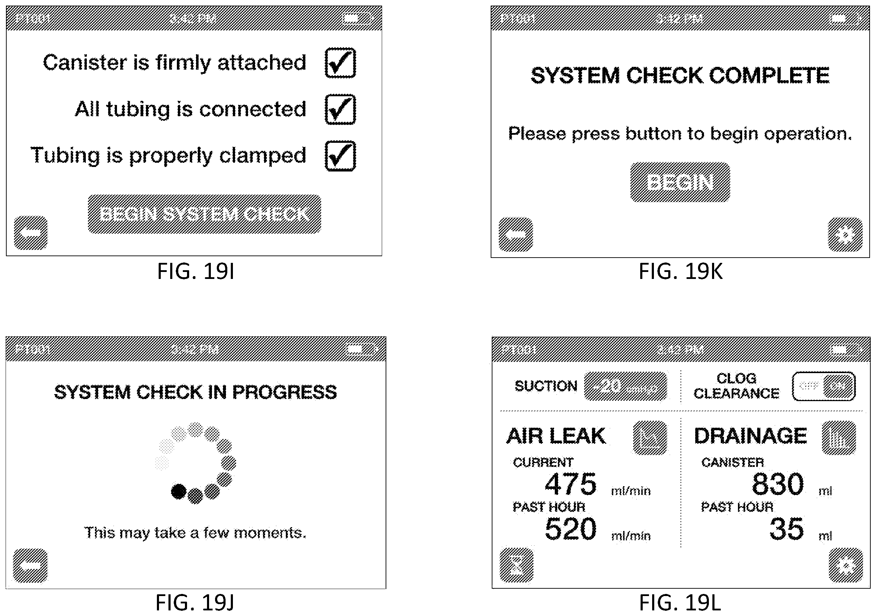

Next, the system prompts the user to complete a self-check, which requires the user to confirm that the canister is firmly attached (Fig. 19F), that all tubing is connected (Fig. 19G), and that the tubing is properly clamped (Fig. 19H). At this point the user can begin the self-check (Fig. 19I). During the self-check, the system may check for presence of the drainage canister via a reflective tab and for airtightness of the system via pressure measurements (Fig. 19J). The self-check process may include the controller pulling suction to a specified level and monitoring changes in pressure to verify air-tightness of the drainage canister, connections of the drainage tubing are properly made, and the seals pneumatically connecting the drainage canister to the controller are secure.

As part of the system initialization and drainage canister replacement processes, the controller may check for the presence and proper connection of a drainage canister before continuing to normal operation. The drainage canister may be equipped with a highly-reflective tab. The controller utilizes a built-in, infrared reflectivity sensor to detect the proper installation of the drainage canister using the reflective tab. The reflectivity of the reflective tab is acutely sensitive to the relative angle of the reflective tab to the sensor and can determine whether the drainage canister is fully seated or not. The controller may verify that the canister attached is an authentic drainage canister.

If the system check is successful, the controller then allows the user to begin using the system (Fig. 19K). At this point, the system's main screen is displayed, which provides information on the suction setting, the status of clog clearance, the amount of air leak (current and over the past hour, or other time frame), and the amount of drainage (in the canister and over the past hour or other time frame) (Fig. 19L).

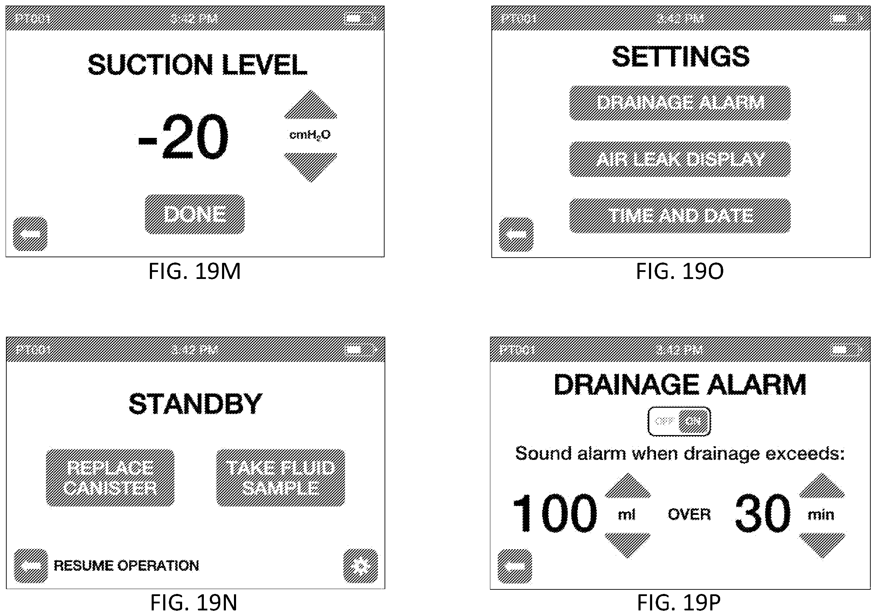

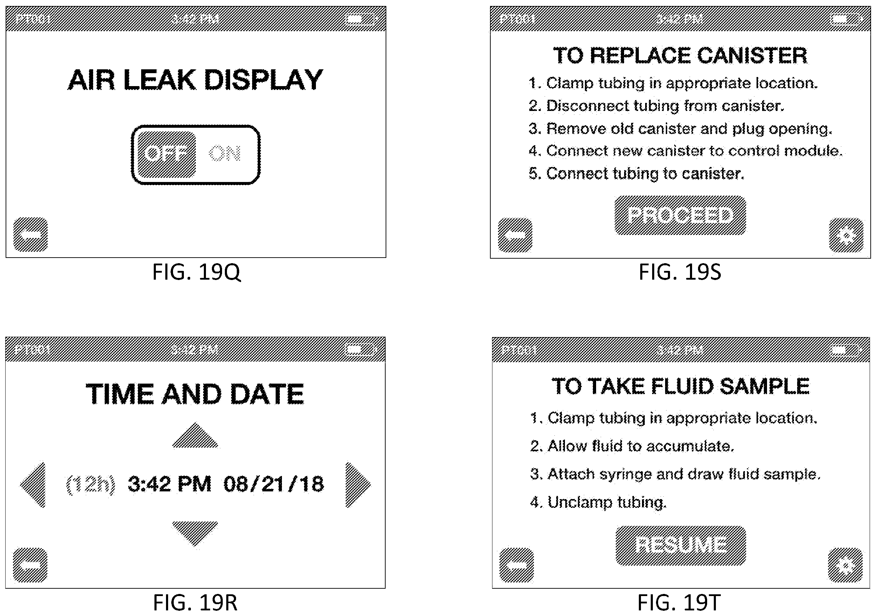

If the user presses the suction level on the main screen, he/she is taken to the suction setting screen, where it can be adjusted (Fig. 19M). Also accessible on the main screen is the standby button (hourglass icon at bottom left). When the user presses this button, he/she is prompted to either resume operation, replace the canister, or take a fluid sample (Fig. 19N). Also accessible on the main screen is the settings button (gear icon at bottom right). When the user presses this button, he/she is prompted to access the drainage alarm, air leak display, or time and date (Fig. 19O). Upon pressing the drainage alarm button, the user can turn the alarm on and off as well as adjust the drainage rate threshold for alarm activation (Fig. 19P). Upon pressing the air leak display button, the user can turn on or off the display of air leak information on the main screen (Fig. 19Q). Upon pressing the time and date button, the user can adjust the time and date of the system (Fig. 19R). When the replace canister button is pressed via the standby screen, the user is prompted to follow the on-screen instructions (Fig. 19S). Similarly, when the fluid sample screen is pressed via the standby screen, the user is prompted to follow the on-screen instructions (Fig. 19T).

In addition, the controller may display a screen and/or sound an alarm when the canister is full if the capacitive sensor detects that the drainage canister has reached its maximum capacity. The controller will alert and prompt the attending physician to replace the canister. If the alert is ignored or an attempt to use a full canister is made, the controller may not allow normal operation until a new, or empty, drainage canister is attached.

In addition, the controller may display a screen and/or sound an alarm if the canister is disconnected from the controller. This may be determined using IR sensor(s) which determine when the drainage canister is no longer connected or not fully connected, to the controller. The controller will alert and/or prompt the attending physician to re-attach the canister. If the alert is ignored or not addressed properly, the controller may not allow normal operation until the drainage canister is properly attached.

In addition, the controller may display a screen and/or sound an alarm if the battery level is below a set threshold. The controller may prompt the user to plug the device into wall power to recharge the battery. If the alert is ignored and the battery level approaches dangerously low levels, the controller may place the system in a safe state before power is lost. The controller may display remaining battery life as a time or percentage. The battery icon may also implement color coding to indicate battery level.

In addition, the controller may display a screen and/or sound an alarm if the air leak rate is above a set threshold. The controller may alert and prompt the attending physician of the leak presence and prompt the physician to check the system for signs of leaks coming from tubing connections, chest tube drainage hole placement, etc. The controller may continue to alert until the air leak is resolved.

In addition, the controller may display a screen and/or sound an alarm if the drainage volume rate has exceeded the acceptable value as defined by the attending physician in the settings. The controller may alert and prompt the attending physician to verify the volume in the drainage canister using physical graduations or other means. The controller may continue to alert until the issue is resolved.

In addition, the controller may display a screen and/or sound an alarm if an obstruction is detected in the chest tube. If the controller attempts to run a clog clearance cycle and the pressure readings at the tube-tube interface exceed the maximum suction threshold, it will alert and prompt the attending physician to check the chest tube for any kinks or obstructions within the tube. The controller may continue to alert until the issue is resolved.

In addition, the controller may display a screen and/or sound an alarm if an obstruction is detected in the drainage line. If the controller attempts to draw suction and the suction level in the drainage canister increases (the pressure becomes more negative) but the suction level at the tube-tube interface doesn't change significantly, it will alert and prompt the attending physician to verify that the tubing clamp is disengaged and check for any kinks or obstructions within the drainage tubing. The controller may continue to alert until the issue is resolved.

In addition, the controller may display a screen and/or sound an alarm if the device is knocked over. If the controller detects a change in position of the system beyond the acceptable range, it will alert and prompt the attending physician to return the device to its upright position. The position detection may be done with accelerometers, a gyroscope, camera, etc. The controller may continue to alert and place the system in a safe state until the issue is resolved.

Also accessible on the main screen are the air leak and drainage volume trend buttons (graph icons next to respective text). When the user presses these buttons, they are taken to screens displaying historical data over the past 6, 12, or 24 hours, depending on the user selection (Figs. 19U-Z). On all screens, the time and battery status may be displayed.

The battery icon is shown with a lightning bolt in its center when it is plugged in, and becomes red when critically low. On screens other than the patient selection screen, the patient ID number may be displayed.

In another embodiment, the system may display an icon or alert when the patient has met predetermined criteria for air leak rate and/or drainage volume that indicate the chest tube is ready to be removed.

### Software

In some embodiments of the system, the location of a clog can be determined to be either in the chest tube or the drainage tubing. When the system attempts to clear a clog in the chest tube, it temporarily increases the level of suction by running the pump. If, after the pump is turned off, this increased suction (which may be measured at the canister) does not attenuate substantially within a set time period, this indicates that the chest tube relief valve has not opened and air has not entered the system. This means that a clog has been detected. As a result of this condition, the controller may open the drainage tube relief valve connected to the relief lumen of the drainage tubing. If the suction (negative pressure) measured in the canister still has not attenuated substantially (become less negative), the controller may determine that the clog is likely in the drainage tubing. If adequate attenuation of the pressure measured in the canister does occur after opening the drainage tube relief valve, the controller may determine that the clog is likely in the chest tube.

In some embodiments, the controller can determine the clog location based on pressures sensed in both the drainage canister and at the tube-tube interface. In these embodiments, suction is increased in order to clear the chest tube. If the suction (negative pressure) measured in the drainage canister increases (becomes more negative) above a set threshold, including but not limited to -40, -60, -80, -100, -120, -140, or -160 cmH2O, while the pressure at the tube-tube junction remains at a lower (less negative) suction value, the controller may determine that the clog is in the drainage tubing. If the canister suction does not differ substantially relative to pressure at the tube-tube junction after increased suction is pulled, and attenuation of suction in the system does not occur after the pump has been turned off or decreased, the controller may determine that the clog is in the chest tube. The controller may also be able to determine what type (size, brand, configuration, etc.) or quantity of chest tube(s) is connected to the system based on the measured pressure(s) within the system.

In some embodiments of the system, the minimum suction necessary to keep up with the patient's air leak rate is used to minimize the differential pressure between the inside and outside of the patient's lung in order to expedite healing of the site of the air leak. In this mode, the user is not required to choose a specific suction level, as the system controller will automatically maintain the minimum level necessary to keep the patient's lung inflated.

In some embodiments, the system may have pre-set recommendations for drainage volume or air leak rates which indicate when it is appropriate for removing the patient's chest tube. The system may indicate when the air leak rate and/or drainage volume values are within acceptable levels, or may instruct the user to remove the patient's chest tube. In some embodiments, these recommendations may be based on the historical trends of data from a specific patient, or from aggregated patient data. These data are not limited to drainage volume and air leak rate data, other data collected, or associated with a patient or patient population, may be used.

### Connectivity and Additional Functionality

Some embodiments of the system may transfer data either to or from the chest drainage system controller via hard wire connection or wirelessly. Wireless connections may include wi-fi, NFC, Bluetooth, cellular transmission, proprietary RF channel, or similar methods. Information transmission may be one-way or two-way. Communication may be with computers including servers, personal devices (phones, tablets, watches, etc.), other medical devices, cloud/remote based software, or electric systems.

Data may include, but are not limited to, any one or more of the following:
Data collected by the system: fluid drainage rate, system status, alarm notifications, air leak rate, system usage levels, system usage duration, battery level, average suction, applied changes, maintenance requirements, other programmed notifications, etc.
Data in other systems: patient health data, patient demographic data, aggregated patient data, etc.

Data may be transmitted actively or passively. Recipients of the data may have the option to view data or make system operating changes or both, either manually or defined by an algorithm. Data transmission may be collated for transmission into different sub-groups based on predefined user groups, access level, or allowable remote inquiry.

In some embodiments, the patient's drainage volume output as measured by the device is transmitted to an infusion pump and/or feeding pump, and the amount of fluids being administered are automatically adjusted accordingly. In this manner, the system may be part of a closed-loop fluid balance system, which may include, for example, systems for measuring fluids such as urine output, fecal output, wound drainage, perspiration, and moisture lost during respiration, and systems for administering fluids, such as infusion pumps and feeding pumps.

In some embodiments, the system is capable of communicating with other control modules, hospital monitoring systems, electronic health records, electronic medical systems, or other devices to either share and display information to physicians (e.g. air leak rate or drainage volume over the past hour), to receive information about the patient to be used for various actions (e.g. heart rate, body temperature, or O2 levels to gain insight on patient stability), or to send information about the patient to other devices for various actions (e.g. drainage volume output to inform autotransfusion machine of necessary input to compensate).

In some embodiments, the controller has the capability of wireless charging, for example but not limited to, exposed electrodes that engage with the charging electrodes of a charging station or dock; integrated wireless charging functionality in bedside or floor mount.

In some embodiments, the system makes use of mechanisms to prevent tampering or re-use of disposable components. In one such embodiment, the system may require authorization via PIN or swiping an RFID enabled badge, for example, in order to enable or disable device settings or functionality. In another embodiment, the system requires a specific set of screen touches in order to unlock the device and modify settings or functionality. The disposable components may become unusable after being removed from the controller, for example but not limited to a break-away latch connector that snaps off when the drainage canister is removed from the controller.

In some embodiments, the system has a "check for air leak" mode in which the system monitors various characteristics, including but not limited to, pump activation and pressure, to help identify the presence of an air leak as a final check before removing the chest tube from the patient. One embodiment of this functionality may include a 1-minute data collection period during which the patient coughs, sits up, or does some other action as cause for an air leak to show up; afterwards, the controller screen may indicate the results, for example but not limited to, a plot of chest pressure over time, pump activation over time, air leak in mL/min over time, or an info screen that displays the results in a text or graphic format.

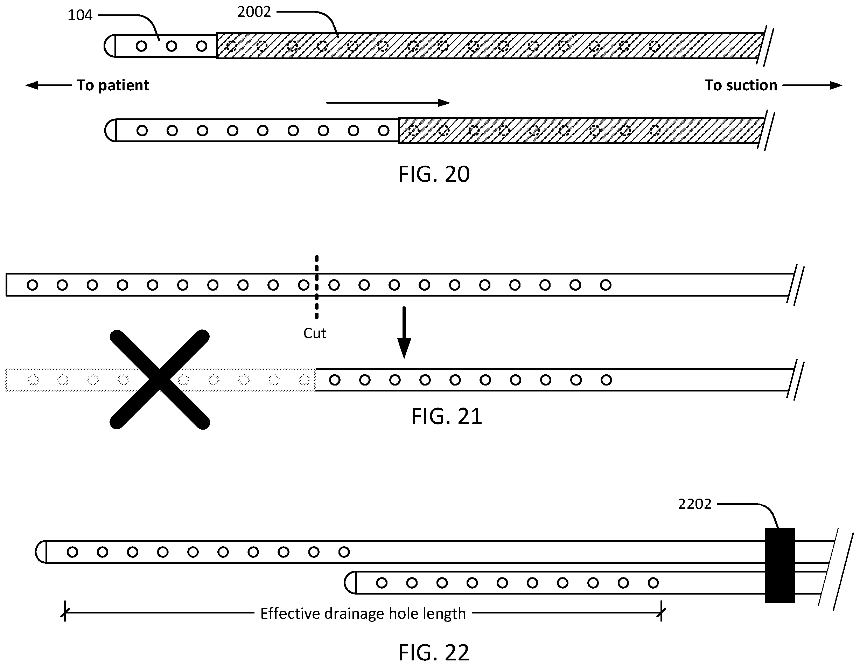

Depending on the physician and/or patient, the desired length of drainage holes or channels may vary; therefore, it may be desirable for the chest tube to have a modular drainage area length to adapt to each clinical situation. In one embodiment of the chest tube, a sheath, such as silicone sheath 2002, is preinstalled, or may be installed by the user, along the length of chest tube 104. The sheath may be moved or removed as desired to expose additional drainage holes as shown in Fig. 20. The sheath may be on the inside or the outside of the chest tube. The method for moving the sheath, or removal of all or part of the sheath, may include: rolling or stretching the chest tube to allow the sheath to move freely around the chest tube, sliding the sheath along the chest tube, cutting the sheath, optionally with a specially designed instrument or tool, tearing the sheath, pulling a suture or thread to change the length of the sheath, etc. The sheath may include weakened sections or objects to facilitate removal.

In another embodiment, the chest tube may come with an excessive drainage area length to allow the physician to cut the drainage area to the desired length, by cutting the chest tube to length. This is shown in Fig. 21. In this embodiment, the system may include a special tool to create the connection hole between the chest tube drainage lumen and the chest tube relief lumen after the chest tube has been cut to length.

In another embodiment of the chest tube, various drainage area lengths may be offered, for example, 4", 5", or 6", less than 4" or longer than 6".

In another embodiment of the chest tube kit, an additional component, such as connecting, or holding, component 2202 is included with the system. Component 2202 holds more than one chest tube together such that the effective hole length is increased, as shown in Fig. 22.

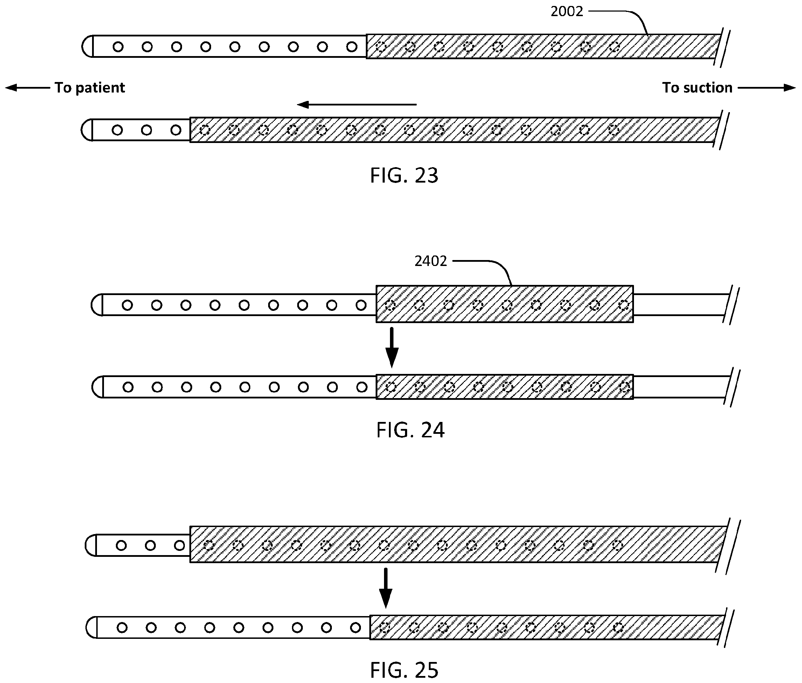

In yet another embodiment of the chest tube, a silicone, or other material, sheath is incorporated at the proximal end of the chest tube and can be pulled toward the distal end (the patient end), or in the opposite direction, to cover or uncover exposed holes, until the desired drainage area length is achieved as shown in Fig. 23.

In another embodiment of the chest tube kit, a silicone, or other material, tape is included to cover drainage holes until the desired drainage area length is achieved.

In yet another embodiment of the chest tube, heat-shrink tubing 2402 may be placed over the undesired drainage areas, as shown in Fig. 24, and shrunk down to cover undesired drainage areas. The heat shrink tubing may or may not shrink in length as it shrinks in diameter. If the heat shrink tubing does shrink in length as heated, it may be used to expose more drainage holes by fixing the tubing at the proximal end, forcing the length of the tubing to decrease as heat is applied, as shown in Fig. 25.

In another embodiment of the chest tube kit, a mandrel and punch are included to allow physicians to punch additional holes as desired. Pad-printed markers may indicate where surgeon-generated hole creation is acceptable and where it should not be cut.

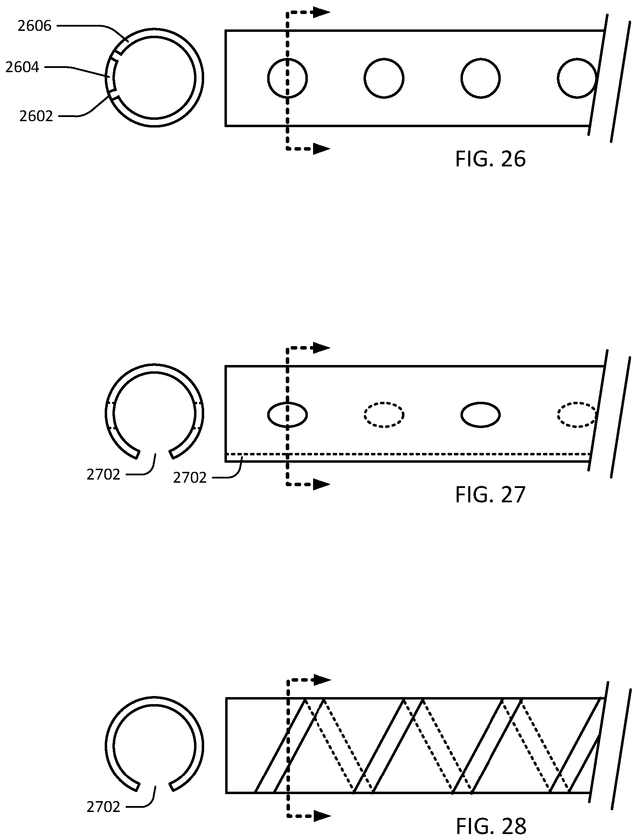

In yet another embodiment of the chest tube, the drainage holes are not completely punched, leaving thin film 2602 attaching hole slug 2604 to chest tube 2606. The physicians would then pull or punch out the desired number of slugs to create an appropriate drainage hole length or number as shown in Fig. 26. The thin film may dissolve in the presence of fluid, such that the appropriate drainage area length is automatically created when the chest tube is placed in the patient.

In another embodiment of the chest tube, drainage area length may be exposed using a silicone-based zipper.

In another embodiment of the chest tube, the dual-lumen extrusion has continuous drainage channel opening 2702 along one or more sides, with additional holes along the sides, as shown in Fig. 27. The chest tube may be cut to the desired length by the physician; a special tool may then be used to create the connection hole between the chest tube drainage lumen and the chest tube relief lumen. In another embodiment, the channel opening pivots back and forth radially along the length of the extrusion to improve drainage area coverage, as shown in Fig. 28.

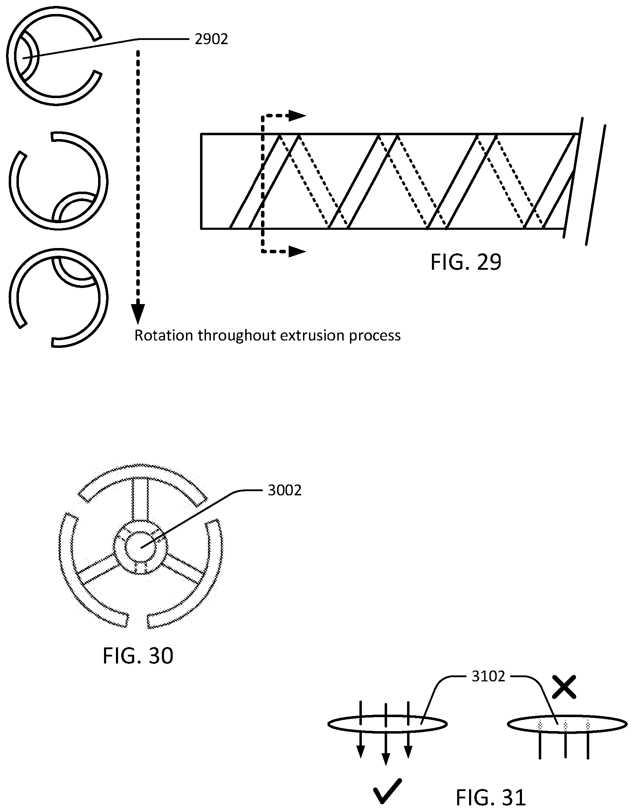

In another embodiment, the chest tube profile (dual-lumen with a channel on the side) is rotated during the extrusion process, so that the drainage area is present in all directions at some point along the extrusion, as shown in Fig. 29. Fig. 29 shows chest tube relief lumen 2902. Note that any of the embodiments disclosed herein, including the embodiments shown in Figs. 20-29, may include a chest tube relief lumen, similar to that shown in Figs. 6A and 6B.

In yet another embodiment of the chest tube, the extrusion consists of a channel drain with independent relief lumen 3002 running down the center, shown in Fig. 30. The chest tube may be cut to the desired length by the physician; a special tool may then be used to create a connection hole between the independent relief lumen and adjacent channels.

In another embodiment of the chest tube, a single channel is cut into the bottom of the extrusion; then twisted axially and heat set to generate a similar result as described above.

In some embodiments, the canister, or other components of the system, may include a hydrophobic, or other suitable, coating to repel body fluids. For example, if the drainage canister gets tipped over or knocked around, blood may coat the inside of the canister and leave a film, creating the potential for drainage volume measurement interference. In one embodiment of the device, a hydrophobic chemical coating may be applied to the inner surface of the drainage canister to repel bodily fluids. The coating may be on the entire canister, or on only certain areas. For example, for example, the coating may be placed on the inner wall nearest to the volume sensing mechanism.

In some embodiments, a thin film may be applied to the canister to repel bodily fluids. The film may comprise polypropylene, polycarbonate, PTFE, Teflon, or other materials. In another embodiment, modified surface finishes may be utilized to prevent blood and other particulate from sticking to the inner surface of the drainage canister. Any of the aforementioned methods for preventing adhesion of blood and other particulate may also be applied to other components of the system, for example, the chest tube, drainage tube, relief valve, and drainage barb.

In some embodiments, anti-foaming mechanisms and/or chemicals may be incorporated into the drainage system, and in particular, into the canister. For example, an anti-foaming additive may be added to the canister to reduce bubbling of drained fluid. In another embodiment, the drainage canister material itself may provide anti-foaming functionality. Hydrophobic and/or oleophobic materials and/or additives may be used for different applications throughout the system. For example, in another embodiment, a blood-repellant barrier may be utilized at the entrance to the drainage tube relief line of the drainage barb to prevent ingress of fluid. The anti-foaming mechanism may be in the form of an anti-foaming tablet, which is incorporated into the canister. The tablet may comprise simethicone, or other anti-foaming ingredient(s).

In another embodiment, a blood-repellant barrier may be incorporated within the drainage canister at the entrance to the suction inlet to act as a protection mechanism to the suction source, in the event of an overfilled canister. In yet another embodiment, blood-repellant barrier 3102 may be incorporated within the drainage canister at the drainage inlet to allow blood and other fluids to pass through into the canister while being repelled from passing through the opposite direction, out of the drainage inlet, as shown in Fig. 31.

In another embodiment of the device, the system may allow for autotransfusion of blood to be re-introduced to the patient. For example, the drainage canister may feature a luer lock valve, stopcock, or other port that can be connected to an autotransfusion machine. This port may also be used to remove contents of the drainage canister during use, if so desired by the physician.

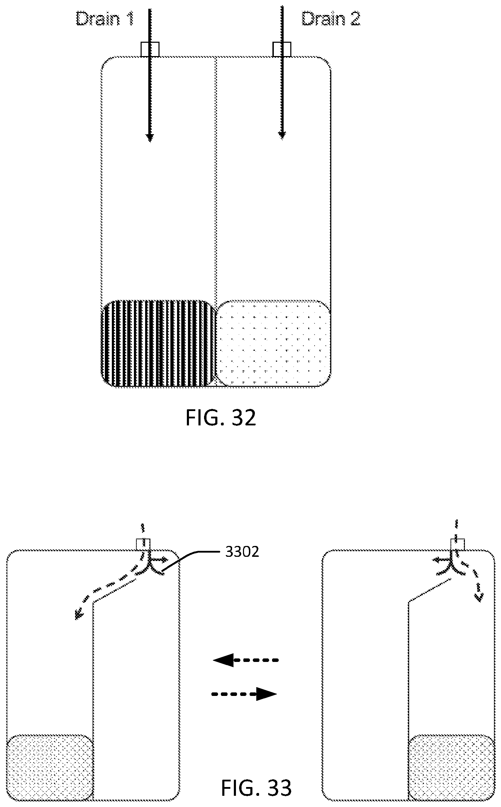

In some embodiments, the drainage canister may consist of a single chamber for collecting the drainage fluid. In some embodiments, one or more rib(s) or support(s) may be added to the drainage canister main body or front plate to provide additional rigidity and strength to the canister. In some embodiments, the drainage canister may comprise two or more separate chambers to collect draining fluid(s) as shown in Fig. 32. The canister may have multiple drainage inlets to be connected in various configurations; for example, one chamber may be connected to a chest tube in the mediastinum, while the other chamber may be connected to a chest tube in the pleural space. Or, for example, both chambers may be connected to chest tubes that reside in the same space but in different locations, so that drainage output based on location of chest tube placement may be investigated. In some embodiments, the system may provide different functions to each chamber independently. For example, one chamber may be set to a suction level of -20cmH2O with clog clearance activated, while the other chamber is set to a suction level of -40cmH2O without clog clearance active.

In some embodiments, the drainage canister may function as either a single chamber or dual chamber device as shown in Fig. 33. The functionality of the canister may be toggled manually by a clinician, for example, using slide lever 3302 to manipulate into which chamber the draining fluid is drained. In a similar embodiment, the functionality of the canister may be toggled through software/hardware by the controller, for example, using a manifold to control the path of fluid drainage.

In yet another embodiment of the drainage canister, the suction port may automatically seal off when disconnected from the controller, for example, by using an umbrella valve, diaphragm valve, or duckbill valve to allow flow out of the canister but not in, making it possible for patient ambulation with the drainage canister alone. In this embodiment, the suction within the canister is maintained even when disconnected from the controller. The drainage tubing remains connected to the canister the entire time, making the use of this functionality simple and easy.

In another embodiment, the drainage tubing may be clamped via a mechanism incorporated into the drainage tubing, such as a valve, switch, or manifold. The clamping mechanism may be internal to, or external to, the drainage tubing. This clamping device may be activated manually by the attending physician or automatically by the controller. In another embodiment of the drainage canister, the drainage canister is offered in a variety of sizes with different volumetric capacities, for example 800mL, 1600mL, and 2000mL.

In some embodiments of the drainage canister, the controller is able to detect specific information about the drainage canister in use, such as total volumetric capacity, relevant features (for example, anti-foaming, hydrophobic, etc.), and patient identification information (to prevent cross-contamination of bodily fluids when used in conjunction with autotransfusion). This information may be provided to the controller by, for example, RFID detection, color detection, or magnetic field detection.

In another embodiment of the drainage canister, data may be stored on the canister (stored to EPROM) in the event that a controller needs to be swapped out, to prevent data loss.

### Alarms

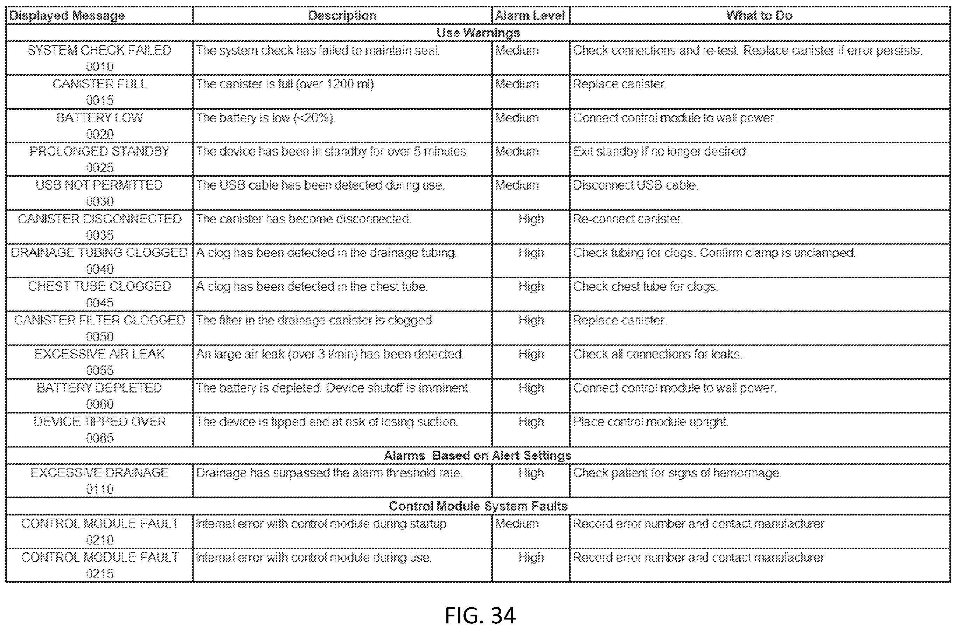

In yet another embodiment, the device has alarms for various conditions that may affect the performance of the device and/or the safety of the patient, such as when the canister is full, the battery is low, or the chest tube is clogged. Some of these alarms and the user actions to be taken to resolve them are shown in Fig. 34.

In some embodiments of the chest drainage system, the monitor provides pulsatile suction (whether via the valve device or via the pump in the monitor to maintain chest tube patency. This suction may be in the form of a sine wave, square wave, or any other suitable oscillatory waveform, and may oscillate between, for example but not limited to 0 to -40 cmH2O, 0 to -60 cmH2O, 0 to -80 cmH2O, 0 to -100 cmH2O, -10 to -40 cmH2O, -20 to -60 cmH2O, and so on. These embodiments may or may not include a chest tube relief lumen.

Some embodiments of the chest drainage system may include a fluid sample port to allow for easy access to draining fluids, for sampling, testing, etc. The port may include a luer-lock valve for the purposes of sampling. The sampling port may be anywhere in the system where drained or draining fluids may be accessed. For example, at the tube-tube junction, along the drainage tube, within the collection chamber, at connection point(s) along the system, etc. The system may provide intuitive, step-by-step sampling instructions via the controller display screen. These instructions may include how to properly clamp the drainage tubing, attach a syringe to the sampling port, and collect a fluid sample. The controller may be placed (or place itself) in standby mode during the sampling process, which may pause normal operation and hold the system in a safe state until the sample is collected.

Some embodiments of the chest drainage system may include an integrated drainage tubing clamp. This claim may slide along all or part of the length of the drainage tubing set and may be pre-installed.

Some embodiments of the chest drainage system may include a positive pressure relief valve. The drainage canister may include an overpressure valve designed to provide an outlet for positively pressurized air to escape the system, for example, when a patient coughs.

Some embodiments of the chest drainage system may include a canister plug for easy disposal of the drainage canister. For example, the drainage canister may have an integrated slot designed to hold a silicone rubber plug, which can be used to close off the drainage canister inlet port after use.

Some embodiments of the chest drainage system may include allow for the use of multiple drainage canisters per patient. Step-by-step instructions may be displayed on the monitor by the controller. These steps may include disconnecting the drainage tubing from the drainage canister, removing and disposing of the drainage canister, installing and attaching a new drainage canister, re-connecting the drainage tubing, etc. During this process, the controller may be placed, or place itself, in standby mode which pauses normal operation and holds the system in a safe state until the drainage canister is replaced.

Some embodiments of the chest drainage system may include physical graduations on the drainage canister, for example, graduation markings on the front face of the canister, ranging from 20 - 1200 ml in increments of 10 mL.

Some embodiments of the chest drainage system may include drainage canister overfill protection. In some embodiments, the drainage canister includes a filter membrane assembly which acts as a physical barrier to prevent fluid from entering the controller in the case of an overfilled drainage canister or if the system is tipped over. The "filter cage" may be a rigid structure that supports a filter membrane (for example but not limited to 0.2, 1.2, or 5 micron pore size) and is located inside the body of the drainage canister. In addition to the physical, filter membrane barrier in the drainage canister, the controller may utilize the built-in capacitive sensor to pre-emptively disable the pump from pulling fluid into the canister, and potentially into the pump of the controller/monitor, when the volume in the drainage canister reaches its capacity. This capacity may be sensed by the controller or preset by the user.

Some embodiments of the chest drainage system may automatically adjust the suction level based on a measured parameter, such as the air leak rate or drainage volume. In some embodiments, the controller may adjust the suction from, for example, -20 cmH2O when the patient has an air leak in excess of, for example, 500 mL/min, and then decrease the suction to a lower level, for example -8 cmH2O, when the air leak diminishes below, for example, 500 mL/min. A similar approach may be taken using drainage volume as the input parameter, whereby the suction level is higher as drainage volume output is higher and then decreases as the patient's drainage output diminishes. These techniques may depend on pre-defined thresholds at which the suction level is adjusted, or may be based on a continuous scale, whereby the suction level is adjusted continuously based on the air leak and/or drainage volume values.

Some embodiments of the chest drainage system may include a battery which allows the system to be portable. For example, the system may include a battery with a 4-hour battery life. Or, for example, the system may include a battery with a 1-hour battery life. Or, for example, the system may include a battery with a 2-hour battery life. Or, for example, the system may include a battery with a 3-hour battery life. Or, for example, the system may include a battery with a 5-hour battery life. Or, for example, the system may include a battery with a 24-hour battery life. Or, for example, the system may include a battery with a greater than 1-hour battery life.

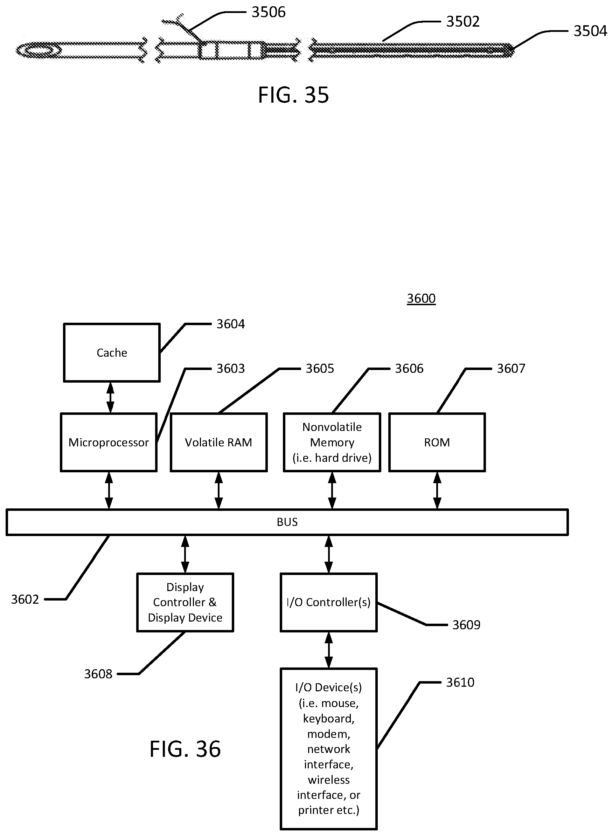

In some embodiments, the system possesses a body contacting, or non-body contacting, sensor system or biological component with a physicochemical detector incorporated into the chest tube (Fig. 35) in which the patient vital signs (e.g. heart rate, respiration rate, blood pressure, body temperature) and/or chemical signals (e.g. analysis of blood, pulmonary fluid) are transmitted to the controller, hospital monitoring systems, and/or other devices to share and display information to physicians, to receive information about the patient to be used for various action, or to send information about the patient to other devices for various actions. Fig. 35 shows chest tube 3502, sensors 3504 and sensor leads or wires 3506 which connect to the controller. The connection may alternatively be wireless.

### Example of Data Processing System

FIG. 36 is a block diagram of a data processing system, which may be used with any embodiment of the invention. For example, the system 3600 may be used as part of a controller/monitor. Note that while FIG. 36 illustrates various components of a computer system, it is not intended to represent any particular architecture or manner of interconnecting the components; as such details are not germane to the present invention. It will also be appreciated that network computers, handheld computers, mobile devices, tablets, cell phones and other data processing systems which have fewer components or perhaps more components may also be used with the present invention.

As shown in FIG. 36, the computer system 3600, which is a form of a data processing system, includes a bus or interconnect 3602 which is coupled to one or more microprocessors 3603 and a ROM 3607, a volatile RAM 3605, and a non-volatile memory 3606. The microprocessor 3603 is coupled to cache memory 3604. The bus 3602 interconnects these various components together and also interconnects these components 3603, 3607, 3605, and 3606 to a display controller and display device 3608, as well as to input/output (I/O) devices 3610, which may be mice, keyboards, modems, network interfaces, printers, and other devices which are well-known in the art.

Typically, the input/output devices 3610 are coupled to the system through input/output controllers 3609. The volatile RAM 3605 is typically implemented as dynamic RAM (DRAM) which requires power continuously in order to refresh or maintain the data in the memory. The non-volatile memory 3606 is typically a magnetic hard drive, a magnetic optical drive, an optical drive, or a DVD RAM or other type of memory system which maintains data even after power is removed from the system. Typically, the non-volatile memory will also be a random access memory, although this is not required.

While FIG. 36 shows that the non-volatile memory is a local device coupled directly to the rest of the components in the data processing system, the present invention may utilize a non-volatile memory which is remote from the system; such as, a network storage device which is coupled to the data processing system through a network interface such as a modem or Ethernet interface. The bus 3602 may include one or more buses connected to each other through various bridges, controllers, and/or adapters, as is well-known in the art. In one embodiment, the I/O controller 3609 includes a USB (Universal Serial Bus) adapter for controlling USB peripherals. Alternatively, I/O controller 3609 may include IEEE-1394 adapter, also known as FireWire adapter, for controlling FireWire devices, SPI (serial peripheral interface), I2C (inter-integrated circuit) or UART (universal asynchronous receiver/transmitter), or any other suitable technology.

Some portions of the preceding detailed descriptions have been presented in terms of algorithms and symbolic representations of operations on data bits within a computer memory. These algorithmic descriptions and representations are the ways used by those skilled in the data processing arts to most effectively convey the substance of their work to others skilled in the art. An algorithm is here, and generally, conceived to be a self-consistent sequence of operations leading to a desired result. The operations are those requiring physical manipulations of physical quantities.

It should be borne in mind, however, that all of these and similar terms are to be associated with the appropriate physical quantities and are merely convenient labels applied to these quantities. Unless specifically stated otherwise as apparent from the above discussion, it is appreciated that throughout the description, discussions utilizing terms such as those set forth in the claims below, refer to the action and processes of a computer system, or similar electronic computing device, that manipulates and transforms data represented as physical (electronic) quantities within the computer system's registers and memories into other data similarly represented as physical quantities within the computer system memories or registers or other such information storage, transmission or display devices.

The techniques shown in the figures can be implemented using code and data stored and executed on one or more electronic devices. Such electronic devices store and communicate (internally and/or with other electronic devices over a network) code and data using computer-readable media, such as non-transitory computer-readable storage media (e.g., magnetic disks; optical disks; random access memory; read only memory; flash memory devices; phase-change memory) and transitory computer-readable transmission media (e.g., electrical, optical, acoustical or other form of propagated signals-such as carrier waves, infrared signals, digital signals).

The processes or methods depicted in the preceding figures may be performed by processing logic that comprises hardware (e.g. circuitry, dedicated logic, etc.), firmware, software (e.g., embodied on a non-transitory computer readable medium), or a combination of both. Although the processes or methods are described above in terms of some sequential operations, it should be appreciated that some of the operations described may be performed in a different order. Moreover, some operations may be performed in parallel rather than sequentially.

## Claims

1. A drainage system, comprising:
a drainage reservoir configurable to be in fluid communication with a chest tube (104) drainage lumen which is also in fluid communication with a chest tube (104) relief lumen;
a chest tube relief valve (112) in fluid communication with the chest tube (104) relief lumen, wherein the chest tube relief valve (112) has a predetermined opening pressure level;
a pump (124) configurable to be in fluid communication with the chest tube (104) drainage lumen;
a pressure sensor (116) configurable to be in communication with the chest tube (104) drainage lumen;
a controller (122) in communication with the pressure sensor (116) and the pump (124), wherein the controller (122) is configured to actuate the pump (124) at a first suction level sufficient to drain a fluid from the chest tube (104) drainage lumen,
**characterized in that** the controller (122) is further configured to actuate the pump (124) at a second suction level which is higher than the first suction level such that an absence of attenuation in the second suction level over time is indicative of an obstruction in a chest tube (104), and wherein the second suction level is higher than the predetermined opening pressure level of the chest tube relief valve (112), and
wherein the controller (122) has a predetermined level of attenuation such that the attenuation in the second suction level rising above the predetermined level of attenuation whereby suction is reduced is indicative of a lack of the obstruction in the chest tube (104).

2. The system of claim 1 further comprising a drainage tube (108) in fluid communication with the chest tube (104) drainage lumen.

3. The system of claim 1 wherein the chest tube (104) is configured to have a passage fluidly coupling the chest tube (104) relief lumen to the chest tube (104) drainage lumen whereby the passage is located within the chest tube (104) so as to be positioned within a patient body.

4. The system of claim 1 wherein the controller (122) is configured to provide an alert when an obstruction is detected.

## Patentansprüche

1. Drainagesystem, umfassend:
ein Drainagereservoir, das so konfiguriert ist, dass es in Fluidverbindung mit einem Drainagelumen eines Thoraxdrains (104) steht, welches ebenfalls in Fluidverbindung mit einem Entlastungslumen des Thoraxdrains (104) steht;
ein Thoraxdrain-Entlastungsventil (112) in Fluidverbindung mit dem Entlastungslumen des Thoraxdrains (104), wobei das Thoraxdrain-Entlastungsventil (112) ein vorbestimmtes Öffnungsdruckniveau aufweist;
eine Pumpe (124), die so konfiguriert ist, dass sie in Fluidverbindung mit dem Drainagelumen des Thoraxdrains (104) steht;
einen Drucksensor (116), der so konfiguriert ist, dass er mit dem Drainagelumen des Thoraxdrains (104) in Verbindung steht;
einen Controller (122) in Verbindung mit dem Drucksensor (116) und der Pumpe (124), wobei der Controller (122) so konfiguriert ist, die Pumpe (124) mit einem ersten Saugpegel zu betätigen, der ausreicht, um eine Flüssigkeit aus dem Drainagelumen des Thoraxdrains (104) zu drainieren,
**dadurch gekennzeichnet, dass** der Controller (122) ferner so konfiguriert ist, die Pumpe (124) mit einem zweiten Saugpegel zu betätigen, der höher ist als der erste Saugpegel, sodass ein Ausbleiben einer Abschwächung des zweiten Saugpegels über die Zeit ein Hinweis auf eine Obstruktion in einem Thoraxdrain (104) ist, und wobei der zweite Saugpegel höher ist als das vorbestimmte Öffnungsdruckniveau des Thoraxdrain-Entlastungsventils (112), und
wobei der Controller (122) ein vorbestimmtes Abschwächungsniveau aufweist, sodass eine Abschwächung des zweiten Saugpegels, die über das vorbestimmte Abschwächungsniveau ansteigt, wodurch der Sog reduziert wird, ein Hinweis auf das Fehlen der Obstruktion im Thoraxdrain (104) ist.

2. System nach Anspruch 1, ferner umfassend einen Drainageschlauch (108) in Fluidverbindung mit dem Drainagelumen des Thoraxdrains (104).

3. System nach Anspruch 1, wobei der Thoraxdrain (104) so konfiguriert ist, dass er einen Durchgang aufweist, der das Entlastungslumen des Thoraxdrains (104) fluidisch mit dem Drainagelumen des Thoraxdrains (104) koppelt, wobei sich der Durchgang innerhalb des Thoraxdrains (104) befindet, sodass er innerhalb eines Patientenkörpers positioniert ist.

4. System nach Anspruch 1, wobei der Controller (122) so konfiguriert ist, bei Erkennung einer Obstruktion eine Warnmeldung auszugeben.

## Revendications

1. Un système de drainage, comprenant :
un réservoir de drainage configuré pour être en communication fluidique avec une lumière de drainage d'un drain thoracique (104), laquelle est également en communication fluidique avec une lumière de décharge du drain thoracique (104) ;
une valve de décharge du drain thoracique (112) en communication fluidique avec la lumière de décharge du drain thoracique (104), la valve de décharge du drain thoracique (112) présentant un niveau de pression d'ouverture prédéterminé ;
une pompe (124) configurée pour être en communication fluidique avec la lumière de drainage du drain thoracique (104) ;
un capteur de pression (116) configuré pour être en communication avec la lumière de drainage du drain thoracique (104) ;
un contrôleur (122) en communication avec le capteur de pression (116) et la pompe (124), le contrôleur (122) étant configuré pour actionner la pompe (124) à un premier niveau d'aspiration suffisant pour drainer un fluide depuis la lumière de drainage du drain thoracique (104),
**caractérisé en ce que** le contrôleur (122) est en outre configuré pour actionner la pompe (124) à un second niveau d'aspiration qui est supérieur au premier niveau d'aspiration, de telle sorte qu'une absence d'atténuation du second niveau d'aspiration au cours du temps est indicative d'une obstruction dans un drain thoracique (104), et dans lequel le second niveau d'aspiration est supérieur au niveau de pression d'ouverture prédéterminé de la valve de décharge du drain thoracique (112), et
dans lequel le contrôleur (122) présente un niveau d'atténuation prédéterminé, de telle sorte qu'une atténuation du second niveau d'aspiration dépassant le niveau d'atténuation prédéterminé, par laquelle l'aspiration est réduite, est indicative de l'absence de l'obstruction dans le drain thoracique (104).

2. Le système selon la revendication 1, comprenant en outre un tube de drainage (108) en communication fluidique avec la lumière de drainage du drain thoracique (104).

3. Le système selon la revendication 1, dans lequel le drain thoracique (104) est configuré pour comporter un passage couplant fluidiquement la lumière de décharge du drain thoracique (104) à la lumière de drainage du drain thoracique (104), le passage étant situé à l'intérieur du drain thoracique (104) de manière à être positionné à l'intérieur du corps d'un patient.

4. Le système selon la revendication 1, dans lequel le contrôleur (122) est configuré pour fournir une alerte lorsqu'une obstruction est détectée.
